(19) Europäisches Patentamt / European Patent Office / Office européen des brevets

(11) **EP 2 061 742 B1**

(12) **EUROPÄISCHE PATENTSCHRIFT**

(45) Veröffentlichungstag und Bekanntmachung des Hinweises auf die Patenterteilung:
**30.09.2015 Patentblatt 2015/40**

(21) Anmeldenummer: **07802811.5**

(22) Anmeldetag: **22.08.2007**

(51) Int Cl.:
*C07C 45/52* (2006.01)        *C07C 47/22* (2006.01)
*C07C 51/25* (2006.01)        *C07C 57/04* (2006.01)
*B01J 8/22* (2006.01)         *B01J 19/32* (2006.01)
*C08F 2/00* (2006.01)         *C08F 2/20* (2006.01)
*C08F 220/06* (2006.01)

(86) Internationale Anmeldenummer:
**PCT/EP2007/058745**

(87) Internationale Veröffentlichungsnummer:
**WO 2008/023040 (28.02.2008 Gazette 2008/09)**

(54) **AUF NACHWACHSENDEN ROHSTOFFEN BASIERENDE ACRYLSÄURE UND WASSERABSORBIERENDE POLYMERGEBILDE SOWIE VERFAHREN ZU DEREN HERSTELLUNG MITTELS DEHYDRATISIERUNG**

ACRYLIC ACID BASED ON RENEWABLE RAW MATERIALS, WATER-ABSORBING POLYMER STRUCTURES, AND METHOD FOR THE PRODUCTION THEREOF BY MEANS OF DEHYDRATION

STRUCTURES POLYMÈRES ABSORBANT L'EAU ET ACIDE ACRYLIQUE À BASE DE MATIÈRES PREMIÈRES RENOUVELABLES ET PROCÉDÉ DE PRODUCTION DE CEUX-CI PAR DÉSHYDRATATION

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **22.08.2006 DE 102006039205**

(43) Veröffentlichungstag der Anmeldung:
**27.05.2009 Patentblatt 2009/22**

(73) Patentinhaber: **Evonik Degussa GmbH**
**45128 Essen (DE)**

(72) Erfinder:
• **BUB, Günther**
  **45772 Marl (DE)**
• **MOSLER, Jürgen**
  **44577 Castrop-Rauxel (DE)**
• **KUPPINGER, Franz-Felix**
  **45768 Marl (DE)**
• **SABBAGH, Andreas**
  **48249 Dülmen (DE)**
• **STOCHNIOL, Guido**
  **45721 Haltern (DE)**
• **SAUER, Jörg**
  **48249 Dülmen (DE)**

• **WINTERBERG, Markus**
  **45711 Datteln (DE)**
• **KNIPPENBERG, Udo**
  **45772 Marl (DE)**
• **LATOSCHINSKI, Günter**
  **45770 Marl (DE)**
• **FURNO, Franck**
  **40545 Düsseldorf (DE)**
• **SCHWÄRTZKE, Thorsten**
  **45772 Marl (DE)**
• **LEISTNER, Dr., Jörg**
  **33378 Rheda-Wiedenbrück (DE)**

(74) Vertreter: **Lang, Arne et al**
**Evonik Degussa GmbH**
**DG-IPM-PAT**
**Bau 1042 / PB 15**
**Paul-Baumann-Strasse 1**
**45764 Marl (DE)**

(56) Entgegenhaltungen:
**EP-A- 0 959 062        WO-A-2006/092272**
**WO-A-2006/136336      DE-A1- 10 138 150**
**DE-C1- 4 238 493        GB-A- 141 057**

- DATABASE WPI Week 200560 Derwent Publications Ltd., London, GB; AN 2005-585659 XP002405550 & JP 2005 213225 A (NIPPON CATALYTIC CHEM IND CO) 11. August 2005 (2005-08-11)

**Beschreibung**

Die vorliegende Erfindung betrifft ein Verfahren zur Herstellung von Acrylsäure, ein Verfahren zur Herstellung von Polymeren durch Polymerisation von Acrylsäure, vorzugsweise zur Herstellung wasserabsorbierender Polymere, die durch dieses Verfahren erhältlichen wasserabsorbierenden Polymere, wasserabsorbierende Polymere, welche zu mindestens 25 Gew.-% auf teilneutralisierter Acrylsäure basieren, einen Verbund, ein Verfahren zur Herstellung eines Verbundes, den durch dieses Verfahren erhältlichen Verbund, die Verwendung von Acrylsäure zur Herstellung wasserabsorbierender Polymergebilde, eine Vorrichtung zur Herstellung von Acrylsäure, ein Verfahren zur Herstellung von Acrylsäure sowie die durch dieses Verfahren erhältliche Acrylsäure.

An die Reinheit von Acrylsäure, welche zur Herstellung polymerer Verbindungen eingesetzt werden, werden hohe Anforderungen gestellt. Dieses gilt insbesondere dann, wenn es sich bei den Polymeren um so genannte Superabsorber handelt, die in Wundauflagen oder Hygieneartikel eingearbeitet werden. Diese Polymere sind in der Lage, wässrige Flüssigkeiten unter Bildung eines Hydrogels zu absorbieren und damit zu binden. Superabsorber finden daher insbesondere in Hygieneartikeln wie Windeln, Inkontinenzeinlagen, Damenbinden und dergleichen zur Absorption von Körperflüssigkeiten Verwendung. Einen umfassenden Überblick über Superabsorber, ihre Anwendung und ihre Herstellung geben F. L. Buchholz und A. T. Graham (Herausgeber) in "Modern Superabsorbent Polymer Technology", Wiley-VCH, New York, 1998.

Zur Herstellung der superabsorbierenden Polymere wird üblicherweise eine Acrylsäure eingesetzt, welche durch katalytische Gasphasenoxidation von Propylen zu Acrolein, das dann in einer weiteren katalytischen Gasphasenoxidation zu Acrylsäure, anschließende Absorption des gasförmigen Reaktionsgemisches in Wasser, Destillation der so erhaltenen wässrigen Acrylsäurelösung unter Erhalt einer Roh-Acrylsäure und weitere Aufreinigung der Roh-Acrylsäure mittels Destillation oder Kristallisation als Rein-Acrylsäure erhalten wird.

JP 2005 213225 A (NIPPON CATALYTIC CHEM IND CO) offenbart ein Verfahren zur Herstellung von Acrylsäure ausgehend von Glycerin, wobei gasförmiges Glycerin im Gemisch mit Stickstoff durch ein mit Katalysator gefülltes Rohr geleitet wird, wobei das Glycerin zu Acrolein dehydriert wird. Das dabei entstandene gasförmige Acrolein wird dann über einen zweiten Katalysator geleitet und zu Acrylsäure oxidiert. Die Acrylsäure wird mit Wasser gequencht.

Ein Nachteil dieses Verfahrens zur Herstellung von Acrylsäure besteht darin, dass das eingesetzte Edukt (Propylen) aus Erdöl und somit aus nicht-nachwachsenden Rohstoffen hergestellt wird, was vor allem im Hinblick auf die zunehmend schwerer und vor allem teurer werdende Erdölgewinnung aus ökonomischen Aspekten vor allem langfristig bedenklich ist.

Herkömmliche Superabsorber weisen auch den Nachteil auf, dass sie, sofern sie nicht zumindest anteilig natürliche Polymere, wie etwa Zellulose, beinhalten, kaum auf nachwachsenden Rohstoffen basieren. Zwar ist es gelungen, viele der in Hygieneartikeln, insbesondere in Wegwerfwindeln, eingesetzten Komponenten aus biologischen Ausgangsstoffen herzustellen, doch ist ein Ersatz der auf vernetzten Polyacrylaten basierenden Superabsorber durch natürliche, superabsorbierende Polymere, wie etwa durch vernetzte, derivatisierte Stärke oder Zellulose, in der Regel mit signifikanten Einbußen hinsichtlich der Absorbereigenschaften verbunden. Dieses führt meist dazu, dass, um auch nur annähernd die gleichen Absorbereigenschaften in einem Hygieneartikel zu erreichen, wesentlich mehr dieser auf natürlichen Polymeren basierenden Absorber eingesetzt werden müssen. Dieses ist nachteilig, weil die Hygieneartikel dadurch voluminöser und schwerer werden, was den Tragekomfort deutlich einschränkt und zu einem größeren Abfallvolumen führt, das neben mehr Deponieraum bzw. Verbrennungsaufwand mehr Transportkapazitäten für die Abfallentsorgung notwendig macht. All dieses wirkt sich nachteilig auf die Umweltverträglichkeit der auf natürlichen Polymeren basierenden Absorber aus.

Der vorliegenden Erfindung lag die Aufgabe zugrunde, die sich aus dem Stand der Technik ergebenden Nachteile abzumildern oder gar zu überwinden.

Es lag der vorliegenden Erfindung die Aufgabe zugrunde, eine Acrylsäureherstellung aufzuzeigen, der von nachwachsenden Rohstoffen ausgeht und Aussichten auf industrielle Anwendung besitzt. In diesem Zusammenhang sind möglichst lange Laufzeiten bei geringen Störungen, die sich beispielsweise aus Feststoffablagerungen wie Zersetzungs- oder Polymerrückstände in den Reaktoren ergeben können, bedeutend.

Eine Aufgabe lag darin, die Menge der sich im Laufe des Betriebs bei der Herstellung von Acrolein bildenden Feststoffe wie Kohle oder polymere Niederschläge zu vermindern und so einen störungsfreieren Betrieb zu erreichen.

Zudem bestand eine Aufgabe der vorliegenden Erfindung darin, neben möglichst langen und störungsfreien Laufzeiten auch hohe Ausbeuten bei guten Selektivitäten zu erzielen.

Es werden auch Polymere, insbesondere Superabsorber, beschrieben, welche einen besonders geringen Gehalt an extrahierbaren, gegebenenfalls toxischen Bestandteilen aufweisen.

Ausserdem werden Polymere, insbesondere Superabsorber, beschrieben, die umweltverträglich sind und dennoch über hervorragende Anwendungseigenschaften verfügen. So galt es insbesondere, Superabsorber mit einer verbesserten Umweltverträglichkeit bei gleich bleibend guten Absorbereigenschaften zu schaffen.

Die Umweltverträglichkeit der die Polymere beinhaltenden Weiterverarbeitungsprodukte, wie Verbunde allgemein und Hygieneartikel im Besonderen, wird verbessert ohne dass die gewünschten Funktionen, wie Saugfähigkeit, Tragekomfort und einfache Herstellbarkeit dieser Weiterverarbeitungsprodukte darunter leiden.

Auch lag der vorliegenden Erfindung die Aufgabe zugrunde, ein Verfahren zur Herstellung derartiger Polymere und der zur deren Herstellung geeigneten Monomere anzugeben.

Zudem lag eine Aufgabe der vorliegenden Erfindung darin, ein Verfahren und eine Vorrichtung zur Herstellung der Monomere bzw. der Polymere vorzuschlagen, die mit einem möglichst geringen Umrüstaufwand in bestehende großtechnische Fertigungsverfahren und -Vorrichtungen integriert werden kann.

Einen Beitrag zur Lösung der erfindungsgemäßen Aufgaben leisten die Hauptund Nebenansprüche, wobei die jeweils davon abhängigen Unteransprüche bevorzugte Ausgestaltungen der vorliegenden Erfindung darstellen.

Einen Beitrag zur Lösung der erfindungsgemäßen Aufgaben leistet ein Verfahren zur Herstellung von Acrylsäure, mindestens die folgenden Schritte aufweisend:

a. Dehydratisieren von Glycerin zu einem Acrolein aufweisenden Dehydratisierungsprodukt;
b. Gasphasenoxidation des Dehydratisierungsprodukts unter Erhalt eines Acrylsäure aufweisenden Monomergases;
c. in Kontakt bringen des Monomergases mit einem Quenchmittel unter Erhalt einer Acrylsäure aufweisenden Quenchphase;
d. Aufarbeiten der Quenchphase unter Erhalt einer Acrylsäure beinhaltenden Monomerphase;

wobei während des Dehydratisierens eine Flüssigphase a1 und eine Gasphase a2 vorliegt, wobei in der Flüssigphase a1 eine Vielzahl von Gasblasen erzeugt werden, wobei das Dehydratisieren mindestens teilweise in der Flüssigphase erfolgt und wobei mindestens ein Teil der Vielzahl von Gasblasen innerhalb der Flüssigphase a1 geleitet oder geteilt wird.

Einen weiteren Beitrag zur Lösung der vorstehenden Aufgaben leistet ein Verfahren zur Herstellung eines Polymers durch Polymerisation von Acrylsäure, mindestens die folgenden Schritte aufweisend:

A. Dehydratisieren von Glycerin zu einem Acrolein aufweisenden Dehydratisierungsprodukt;
B. Gasphasenoxidation des Dehydratisierungsprodukts unter Erhalt eines Acrylsäure aufweisenden Monomergas;
C. in Kontakt bringen des Monomergas mit einem Quenchmittel unter Erhalt einer Acrylsäure aufweisenden Quenchphase;
D. Aufarbeiten der Quenchphase unter Erhalt einer Acrylsäure beinhaltenden Monomerphase;
E. Polymerisation, vorzugsweise radikalische Polymerisation, der Monomerphase;

wobei während des Dehydratisierens eine Flüssigphase a1 und eine Gasphase a2 vorliegt, wobei in der Flüssigphase a1 eine Vielzahl von Gasblasen erzeugt werden, wobei das Dehydratisieren mindestens teilweise in der Flüssigphase erfolgt und wobei mindestens ein Teil der Vielzahl von Gasblasen innerhalb der Flüssigphase a1 geleitet oder geteilt wird.

Es ist in den erfindungsgemäßen Verfahren bevorzugt, dass mindestens ein, vorzugsweise mindestens zwei der vorstehenden Schritte kontinuierlich erfolgen und nicht durch chargenweise Umsetzungen fortlaufend unterbrochen und wieder angefahren werden müssen. Bevorzugt erfolgen die mindestens die Schritte a. bzw. A. und b. bzw. B. und besonders bevorzugt alle Schritte kontinuierlich.

Allgemein können die Gasblasen in der Flüssigphase durch jede dem Fachmann bekannte Maßnahme erzeugt werden. In einer Ausgestaltung weisen die Gasblasen eine Größe in einem Bereich von 0,01 bis 5 mm, vorzugsweise in einem Bereich von 0,05 bis 1,5 mm und darüber hinaus bevorzugt in einem Bereich von 0,1 bis 1,2 mm auf. Die Blasengröße wird als Mittelwert einer Eindüsung des in der Dehydratisierung verwendeten Blasenerzeugers in Wasser bei Normaldurck bestimmt. Hierzu werden durch eine in dem Reaktor vorgesehen Glasscheibe mindestens 10 Momentaufnahmen von einem 10 x 10 cm großen Bereich der mit Gasblasen durchströmten Flüssigphase a1 in einem Abstand von 20cm vom Blasenerzeuger angefertigt und die Durchmesser der einzelnen auf diesen Aufnahmen abgebildeten Gasblasen bestimmt und die Summe der Durchmesser durch die Zahl der vermessenen Gasblasen geteilt.

In einer Ausgestaltung des erfindungsgemäßen Verfahrens wird mindestens ein Teil der Vielzahl von Gasblasen innerhalb der Flüssigphase a1 geleitet. Hierbei soll die Leitung nicht nur durch an den äußeren Begrenzungen der Flüssigphase a1 sondern auch innerhalb des sich durch die Begrenzung der Flüssigphase ergebenden Raumes erfolgen. Im Zusammenhang mit dem Leiten der Gasblasen ist es vorteilhaft, dass mindestens ein Teil der Gasblasen, vorzugsweise mindestens 30 % und besonders bevorzugt mindestens 70 % dieser Gasblasen sich in Ihrem jeweiligen Volumen nicht vergrößern.

Nach einer weiteren Ausgestaltung des erfindungsgemäßen Verfahrens wird mindestens ein Teil der Vielzahl von Gasblasen innerhalb der Flüssigphase a1 geteilt. Diese Teilung kann beispielesweise dadurch erfolgen, dass aus ursprünglich einer Gasblase zwei, drei oder mehr Gasblasen entstehen, die in Ihrem Volumen kleiner sind als die Gasblase aus der diese hervorgehen.

Ferner wird nach einer anderen Ausgestaltung des erfindungsgemäßen Verfahrens mindestens ein Teil der Vielzahl von Gasblasen innerhalb der Flüssigphase a1 zusätzlich zu einer Verlangsamung durch die Flüssigphase a1 gebremst. Im Allgemeinen hängt die Wanderungsgeschwindigkeit von Gasblasen in einer Flüssigkeit, neben anderen Faktoren, vor allem von der Viskosität der Flüssigkeit ab. Erfindungsgemäß werden geeignete Maßnahmen ergriffen, die dazu führen, dass die Gasblasen bei Ihrem Aufsteigen durch die Flüssigphase a1 zusätzlich durch diese Maßnahmen abgebremst werden. Den Grad der Abbremsung der Gasblasen durch derartige in der Flüssigphase a1 vorgesehene Maßnahmen kann dadurch bestimmt werden, indem unter ansonsten identischen Bedingungen, zunächst Gasblasen durch die Flüssigkeit und in einer weiteren Untersuchung durch die mit der Maßnahme versehene Flüssigkeit geleitet werden. Somit kann der das Aufsteigen der Gasblasen bremsende Einfluss neben der durch die Flüssigphase a1 ohnehin erfolgenden Durchwanderungsgeschwindigkeit der Gasblasen bestimmt werden.

Als geeignete Maßnahmen zur Leitung, Teilung oder Abbremsung oder auch einer Kombination aus mindestens zwei davon, kommen grundsätzlich alle dem Fachmann bekannten und als geeignet scheinenden Maßnahmen in betracht. Eine dieser Maßnahmen stellen in der Flüssigphase a1 befindliche durchströmbare Einbauten dar. Diese Einbauten können sowohl durch die Flüssigphase a1 als auch von den Gasblasen durchströmt werden. Durch geeignet ausgewählte Maßnahmen kann die Wanderungsgeschwindigkeit der Gasblasen durch die Flüssigphase a1 eingestellt und variiert werden. Diese Einbauten können sowohl durch die Flüssigphase a1 als auch von den Gasblasen durchströmt werden. Bevorzugt für das erfindungsgemäßer Verfahren ist es, dass die Wanderungsgeschwindigkeiten der Gasblasen in einem Bereich von 0,01 bis 10 m/s, vorzugsweise in einem Bereich von 0,1 bis 5 m/s und besonders bevorzugt in einem Bereich von 0,1bis 2,5 m/s liegen. Die Wanderungsgeschwindigkeit der Gasblasen wird als durchschnittliche Wanderungsgeschwindigkeit angegeben. Diese kann durch das Erzeugen entsprechender Gasblasen in einem gläsernen, die Flüssigphase a1 beinhaltenden Glaszylinder durch bestimmen von Einzelwanderungsgeschwindigkeiten von 100 Gasblasen durch die Summe der Einzelwanderungsgeschwindigkeiten geteilt durch die Anzahl der Gasblasen bestimmt werden.

Die vorstehend genannten Maßnahmen zur Kontrolle der Verweilzeit der Gasblasen dienen dazu, neben einer möglichst hohen Acrolein-Sättigung in den Gasblasen auch einen möglichst hohen Acroleinaustrag aus dem Dehydratisierungsreaktor zu erzielen. Weiterhin ist es oftmals wünschenswert, dass die Einbauten zu einer Verringerung der Rückvermischung innerhalb des Dehydratisierungsreaktors im Betrieb beitragen. Hierzu tragen die vorstehend genannten Maßnahmen für sich und in einer Kombination aus mindestens zwei davon bei. Es kann in diesem Zusammenhang und in Abhängigkeit von der baulichen Ausgestaltung des Dehydratisierungsreaktors wünschenswert sein, zwei oder mehr gegenläufig wirkende Maßnahmen miteinander zu kombinieren.

In einer Ausgestaltung der vorliegenden Erfindung erfolgt die Erzeugung der Gasblasen durch Zuführen eines Inertgases in die Flüssigphase a1. Als Inertgas kommen grundsätzlich alle dem Fachmann bekannten in Betracht. Das Inertgas sollte so ausgewählt sein, dass es mit den an der Dehydratisierung beteiligten Chemikalien kaum oder besser noch gar nicht reagiert. Weiterhin sollte bei der Auswahl berücksichtig werden, dass das Gas oder die Gase für das Gasblasengas oder Zusatzgas das Dehydratisierungsprodukt und vorzugsweise Acrolein gut aufnehmen können. Als Gase für die Erzeugung von Gasblasen kommen vorzugsweise Gase mit einem Gehalt von mindestens 10 Vol.-%, vorzugsweise mindestens 50 Vol.-% und besonders bevorzugt mindestens 80 Vol.-%, jeweils bezogen auf das Zusatzgas, Luft, $CO_2$, $N_2$ oder Wasserdampf oder einer Mischung aus mindestens zwei davon und besonders bevorzugt $N_2$ und Wasserdampf und darüber hinaus bevorzugt $N_2$ in betracht. Es ist bevorzugt, dass möglichst viel der Flüssigphase a1 von Gasblasen durchströmt werden. So kann erreicht werden, dass das Dehydratisierungsprodukt aus der Flüssigphase a1 in die Gasphase a2 überführt wird. Dieses kann durch einen auch als "Stripping" bezeichneten Vorgang erfolgen. Danach wird das Dehydratisierungsprodukt durch das auch als Schleppgas zu bezeichnende Gas in den Gasblasen aufgenommen und aus der Flüssigphase a1 in die Gasphase a2 überführt und - sofern erforderlich - das Dehydratisierungsprodukt wieder verflüssigt. So wird erreicht, dass die Konzentration an Dehydratisierungsprodukt in der Gasphase höher ist als in der Flüssigphase. Es ist durchaus bevorzugt, dass der Gehalt an Glycerin in der Gasphase um das mindestens 1,5-Fache, vorzugsweise mindestens das 3-Fache und besonders bevorzugt um mindestens das 5-Fache über dem der Flüssigphase liegt.

Durch die Aufnahme des Dehydratisierungsprodukts in die Gasblasen und dadurch, dass die Gasblasen schnellstmöglich die Flüssigphase a1 verlassen, wird ein möglichst rückvermischungsarmer Austrag des Dehydratisierungsprodukts aus der Flüssigphase a1 erreicht. So wird durch die Vielzahl der Gasblasen das bei der Dehydratisierung entstandene Acrolein aus der Flüssigphase a1 in die Gasphase a2 überführt. Dieses kann noch im gasförmigen Zustand der Gasphasenoxidation zugeführt werden. Im Allgemeinen erfolgt dieses nach einer Reaktorverweilzeit (Verweilzeit = Volumenstrom / Reaktorvolumen) in einem Bereich von 1 bis 30 Minuten, vorzugsweise in einem Bereich von 2 bis 20 Minuten und besonders bevorzugt in einem Bereich von 5 bis 15 Minuten.

In einer Ausgestaltung des erfindungsgemäßen Verfahrens ist bevorzugt, dass das Glycerin aus der Verseifung von Fetten gewonnen wird. Bei diesen Fetten kann es sich sowohl um tierische als auch um pflanzliche Fette handeln. Tierische Fette fallen insbesondere bei der Tierkörperverwertung an. Pflanzliche Fette fallen in großen Mangen bei der Ölgewinnung aus Ölfrüchten wie Raps, Soja, Sesam, Oliven und Sonnenblumenkernen an. Große Mengen von Glycerin fallen insbesondere bei der Herstellung von sogenanntem "Biodiesel" aus Rapsöl an, wie WO-A-2004/029016 unter anderem zu entnehmen ist. Folglich ist es in dem erfindungsgemäßen Verfahren bevorzugt, dass das Glycerin bei der Erzeugung von flüssigen Brennstoffen aus natürlichen Rohstoffen anfällt. Dieses ist insbesondere bei Ölmühlen nachgeschalteten Verseifungseinrichtungen gegeben.

In dem erfindungsgemäßen Verfahren ist es weiterhin bevorzugt, dass die Dehydratisierung entlang einer Strecke erfolgt, über die eine Glycerinkonzentration abfällt. Als Strecke kommt hier in der Regel die Längsrichtung des zur Dehydratisierung eingesetzte Reaktors in Betracht. Im allgemeinen beginnt die Strecke an dem Eintritte des Edukts in den Reaktor und endet mit dem Produktauslass des Reaktors. So ist es in dem erfindungsgemäßen Verfahren weiterhin bevorzugt, dass entlang dieser Strecke eine Druckveränderung erfolgt. In einigen Fällen ist der Druck am Edukteintritt höher als der Druck am Produktaustritt. Der Edukteintrittsdruck ist in diesen Fällen vorzugsweise in einen Bereich von 1 bis 300 mbar, bevorzugt in einen Bereich von 10 bis 200 mbar und besonders bevorzugt in einen Bereich von 20 bis 120 mbar höher als der Druck am Produktaustritt. Es ist weiterhin in dem erfindungsgemäßen Verfahren bevorzugt, dass die Dehydratisierung entlang einer Strecke, über die eine Glycerinkonzentration abfällt, erfolgt, wobei es bevorzugt ist, dass entlang dieser Strecke unterschiedliche Strömungsgeschwindigkeiten bestehen. So ist es bevorzugt, dass die Strömungsgeschwindigkeit am Edukteintritt geringer ist als am Produktaustritt. Die vorstehenden Maßnahmen sind für den kontinuierlichen Betrieb des erfindungsgemäßen Verfahrens vorteilhaft.

Die Dehydratisierung erfolgte mindestens teilweise in flüssiger Phase. Als flüssige Phasen sind insbesondere wässrige Systeme bevorzugt. Wenn die Dehydratisierung wenigstens teilweise oder gar vollständig in einer flüssigen Phase durchgeführt wird, hat dieses, insbesondere wenn dies eine wässrige Phase ist, den Vorteil, dass bei hohen Glycerinkonzentrationen hohe Acroleinkonzentrationen in der wässrigen Phase erreicht werden können, die durch die Gasblasen schnellstmöglich ausgetragen werden können. Diese wässrigen Phasen mit hohen Acroleinkonzentrationen können direkt in dem nächsten Schritt der Gasphasenoxidation eingesetzt werden. Ein weiterer Vorteil der Flüssigphasendehydratisierung besteht darin, dass durch die flüssige Phase eine Spülwirkung erzielt werden kann, mit der eine Belagbildung auf im Reaktor deutlich reduziert werden kann, was zu höheren Reaktorlaufzeiten und damit zu einem geringeren Regenerierungsbedarf des Reaktors führt.

Im Allgemeinen kann die Dehydratisierung in einer Temperatur in einem Bereich von 100 bis 400°C, vorzugsweise in einem Bereich von 130 bis 350°C und besonders bevorzugt in einem Bereich von 150 bis 330°C erfolgen. Ein weiterer Vorteil der Flüssigphasendehydratisierung besteht darin, dass diese bei verhältnismäßig moderaten Temperaturen, in einem Bereich von 160 bis 310°C, bevorzugt in einem Bereich von 200 bis 300°C und darüber hinaus bevorzugt in einem Bereich von 250 bis 290°C durchgeführt werden kann. Diese Temperaturbereiche liegen bei erhöhten Druckverhältnissen meist deutlich unter der bei Normaldruck bestimmten Zersetzungs- und Siedetemperatur des Glycerins von etwa 290°C, was zur Verringerung von Zersetzungsrückständen und Polymeren sowie anderen Verunreinigungen führt, die sich nachteilig auf die Betriebsdauer der Gasphasenoxidation auswirken. Bei der Flüssigphasendehydratisierung ist es bevorzugt, dass diese in einer Kreisfahrweise erfolgt, bei der die glycerinhaltige flüssige Phase durch eine Pumpe den als ein Drucksystem ausgestalteten und Katalysator aufweisenden Reaktor geführt wird. So lassen sich auf eine schonende Weise neben einer hohen Selektivität höhere Umsätze und deutlich weniger Nebenprodukte erhalten.

In einer anderen Ausgestaltung des erfindungsgemäßen Verfahrens ist es bevorzugt, dass zumindest das zum Erzeugen der Gasblasen in dem Reaktor eingesetzte Gas nach verlassen des Reaktors mindestens teilweise, beispielsweise zu mindestens 1 Vol.-%, vorzugsweise zu mindestens 10 Vol.-% und besonders bevorzugt mindesten 30 Vol.-%, wieder in den Reaktor zum erneuten Erzeugen von Gasblasen eingespeist wird. Dieses kann durch eine Kreisgasfahrweise erfolgen, bei der das Gas nach mindestens teilweiser Abtrennung des Acroleins als Dehydratisierungsprodukt wieder in den Dehydratisierungsreaktor eingespeist wird. Getrennt oder auch zusammen mit diesem Gas kann wenigstens ein Teil, beispielsweise zu mindestens 1 Vol.-%, vorzugsweise zu mindestens 10 Vol.-% und besonders bevorzugt mindesten 30 Vol.-%, des nicht in der Dehydratisierung umgesetzten Glycerins wieder in den Dehydratisierungsreaktor eingespeist werden. Zudem ist es bevorzugt, dass auch die mindestens teilweise von dem Dehydratisierungsprodukt befreite wässrige Phase mindestens teilweise, beispielsweise zu mindestens 1 Vol.-%, vorzugsweise zu mindestens 10 Vol.-% und besonders bevorzugt mindesten 30 Vol.-%, wieder in den Dehydratisierungsreaktor eingespeist und somit im Kreis gefahren wird. Weiterhin können die vorstehend geschilderten Rückführungen in den Reaktor für sich oder in Kombination von mindestens zwei erfolgen, wobei die Durchführung aller drei vorstehend beschriebener Rückführungen bevorzugt ist.

In einer weiteren Ausgestaltung des erfindungsgemäßen Verfahrens ist es zudem auch möglich, dass die die Dehydratisierung teilweise in einer Gasphase erfolgt. Die Dehydratisierung in Gasphase hat sich insbesondere bei der Umsetzung von Glycerin aus der Fettverseifung bewährt. Dieses Glycerin führt in der Regel eine hohe Salzfracht mit sich, die durch den Verdampfungsschritt der Gasphasendehydratisierung recht gut abgetrennt werden kann. Wie auch bei der Flüssigphasendehydratisierung ist es auch bei der Gasphasendehydratisierung bevorzugt, dass diese in Gegenwart von Wasser erfolgt.

Folglich ist es in dem erfindungsgemäßen Verfahren bevorzugt, dass das Glycerin in einer wässrigen Phase eingesetzt wird. Im Fall der Flüssigphasendehydratisierung weist diese flüssige Glycerinphase im allgemeinen einen Wassergehalt in Bereich von 0 bis 30 Gew.-%, vorzugsweise in einem Bereich von 0 bis 20 Gew.% und besonders bevorzugt in einem Bereich von 0 bis 10 Gew.-% Wasser, jeweils bezogen auf die wässrige Phase, auf. Im Fall der Gasphasendehydratisierung weist die wässrige Glycerinphase im Allgemeinen eine Wassermenge in einem Bereich von mehr als 30 bis 97 Gew.-%, vorzugsweise in einem Bereich von 60 bis 95 Gew.-% und darüber hinaus bevorzugt in einem Bereich von 70 bis 90 Gew.-%, jeweils bezogen auf die wässrige Glycerinphase, auf. Der weitere Hauptbestandteil der Glycerinphase ist Glycerin.

Die Dehydratisierung kann grundsätzlich bei jedem dem Fachmann geeignet erscheinenden Drücken erfolgen. Es ist jedoch bevorzugt, dass die Dehydratisierung bei einem Druck in einem Bereich von 2 bis 200 bar, vorzugsweise 10 bis 150 bar und besonders bevorzugt in einem Bereich von 15 bis 70 bar erfolgt. Es ist weiterhin vorteilhaft, bestimmte Temperatur- und Druckbereich bei der Dehydratisierung einzuhalten.

Nach einer anderen Ausgestaltung des erfindungsgemäßen Verfahrens ist es bevorzugt, Flüssigphasendehydratisierung und Gasphasendehydratisierung miteinander zu kombinieren. Nach einer Form des erfindungsgemäßen Verfahrens kann das Glycerin zunächst der Gasphasendehydratisierung und anschließend der Flüssigphasendehydratisierung oder in umgekehrter Reihenfolge zugeführt werden. Bei der erstgenannten Abfolge besteht der Vorteil, dass aus der Fettverseifung stammende, stark mit Salz befrachtete Glycerinchargen zunächst durch eine Verdampfung in der Gasphasendehydratisierung von dieser Salzfracht bereit werden können um dann anschließend in der Flüssigphasendehydratisierung durch die Kreisfahrweise weiter zu hohen Ausbeuten und Selektivitäten mit wenigen Nebenprodukten umgesetzt werden.

Nach einer weiteren Ausgestaltung des erfindungsgemäßen Verfahrens wird bei diesem ein Dehydratisierungskatalysator eingesetzt. Als Dehydratisierungskatalysatoren kommen sowohl saure als auch alkalische Katalysatoren in Betracht. Saure Katalysatoren sind insbesondere wegen der geringen Neigung zur Oligomerenbildung bevorzugt. Der Dehydratisierungskatalysator kann sowohl als homogener als auch als heterogener Katalysator eingesetzt werden. Wenn der Dehydratisierungskatalysator als heterogener Katalysator vorliegt, ist es bevorzugt, dass der Dehydratisierungskatalysator mit einem Träger x. in Kontakt steht. Als Träger x. kommen alle dem Fachmann als geeignet erscheinenden Feststoffe in Betracht. In diesem Zusammenhang ist es bevorzugt, dass diese Feststoffe geeignete Porenvolumina aufweisen, die zur guten Anbindung und Aufnahme des Dehydratisierungskatalysators geeignet sind. Außerdem sind Gesamtporenvolumen nach DIN 66133 in einem Bereich von 0,01 bis 3 ml/g bevorzugt und in einem Bereich von 0,1 bis 1,5 ml/g besonders bevorzugt. Zudem ist es bevorzugt, dass die als Träger x. geeigneten Feststoffe eine Oberfläche in einem Bereich von 0,001 bis 1000 $m^2$/g, vorzugsweise in einem Bereich von 0,005 bis 450 $m^2$/g und darüber hinaus bevorzugt in einem Bereich von 0,01 bis 300 $m^2$/g nach BET-Test gemäß DIN 66131 aufweisen. Als Träger für den Dehydratisierungskatalysator kann zum einen ein Schüttgut, das einen mittleren Teilchendurchmesser in einem Bereich von 0,1 bis 40 mm, vorzugsweise in einem Bereich von 1 bis 10 mm und darüber hinaus bevorzugt in einem Bereich von 1,5 bis 5 mm aufweist, eingesetzt werden. Ferner kann die Wand des Dehydratisierungsreaktors als Träger dienen. Weiterhin kann der Träger an sich sauer oder basisch sein oder ein saurer oder basischer Dehydratisierungskatalysator auf einen inerten Träger aufgebracht werden. Als Aufbringtechniken sind insbesondere Eintauchen bzw. Imprägnieren oder das Einarbeiten in eine Trägermatrix zu nennen.

Als Träger x., die auch Dehydratisierungskatalysatoreigenschaften aufweisen können, eignen sich insbesondere natürliche oder synthetische silikatische Stoffe, wie insbesondere Mordenit, Montmorillonit, saure Zeolithe; mit ein-, zwei oder mehrbasigen anorganischen Säuren, insbesondere Phosphorsäure, oder saure Salze anorganischer Säuren belegte Trägerstoffe, wie oxidische oder silikatische Stoffe, beispielsweise $Al_2O_3$, $TiO_2$; Oxide und Mischoxide, wie beispielsweise Gamma-$Al_2O_3$ und ZnO-$Al_2O_3$- Mischoxide der Heteropolysäuren.

Es entspricht einer erfindungsgemäßen Ausführungsform, dass der Träger x. mindestens teilweise aus einer oxidischen Verbindung besteht. Derartige oxidische Verbindungen sollten mindestens eines der Elemente aus Si, Ti, Zr, Al, P oder eine Kombination von mindestens zwei davon aufweisen. Derartige Träger können auch selber durch ihre sauren bzw. basischen Eigenschaften als Dehydratisierungskatalysator wirken. Eine bevorzugte sowohl als Träger als x. als auch als Dehydratisierungskatalysator wirkende Verbindungsklasse beinhalten Silicium-,Aluminium-, Phosphoroxide. Bevorzugte basische sowohl als Dehydratisierungskatalysator als auch als Träger x. fungierende Stoffe beinhalten Alkali, Erdalkali, Lanthan, Lanthanoide oder eine Kombination von mindestens zwei davon in ihrer oxidischen Form. Derartige saure oder basische Dehydratisierungskatalysatoren sind sowohl bei der Degussa AG als auch bei der Südchemie AG kommerziell erhältlich. Eine weitere Klasse stellen Ionenaustauscher dar. Auch diese können sowohl in basischer als auch in saurer Form vorliegen.

Als homogene Dehydratisierungskatalysatoren kommen insbesondere anorganische Säuren, vorzugsweise Phosphor beinhaltende Säuren und darüber hinaus bevorzugt Phosphorsäure in Betracht. Diese anorganischen Säuren können auf dem Träger x. durch Eintauchen bzw. Imprägnieren immobilisiert werden. Eine andere Gruppe interessanter homogener Katalysatoren sind schwefelhaltige Säuren wie Schwefelige Säure oder Schwefelsäure oder eine Mischung davon.

Insbesondere bei der Gasphasendehydratisierung hat sich der Einsatz von heterogenen Katalysatoren besonders bewährt. Bei der Flüssigphasendehydratisierung werden jedoch sowohl homogene als auch heterogene Dehydratisierungskatalysatoren eingesetzt.

Zudem ist es bevorzugt, dass in dem erfindungsgemäßen Verfahren ein Dehydratisierungskatalysator mit

einem $H_0$-Wert in einem Bereich von +1 bis -10, vorzugsweise in einem Bereich von +2 bis -8,2 und darüber hinaus bevorzugt bei der Flüssigphasendehydratisierung in einem Bereich von +2 bis -3 und in der Gasphasendehydratisierung in einem Bereich von -3 bis -8,2 eingesetzt wird. Der $H_0$-Wert entspricht der Säurefunktion nach Hammett und lässt sich durch die sogenannte Amintritation und Verwendung von Indikatoren oder durch Absorption einer gasförmigen Base ermitteln - siehe "Studies in Surface Science and Catalytics", vol. 51, 1989: "New solid Acids and Bases, their catalytic Properties", K. Tannabe et al. Weitere Einzelheiten zur Herstellung von Acrolein aus Glycerin sind weiterhin DE 42 38 493 C1 zu entnehmen.

In einer weiteren Ausgestaltung des erfindungsgemäßen Verfahrens erfolgt die Gasphasenoxidation im Schritt b) des erfindungsgemäßen Verfahrens in Gegenwart eines oder mehrerer Oxidationskatalysatoren, welche Übergangsmetalle in elementarer oder in chemisch gebundener Form oder beides aufweisen. Bei den Oxidationskatalysatoren ist es bevorzugt, dass diese wenigstens eines der Elemente Molybdän, Wolfram oder eine Kombination aus mindestens zwei davon in mindestens teiloxidischer Form aufweisen. Derartige Oxidationskatalysatoren werden vorzugsweise als heterogene Katalysatoren mit einem Träger y. in Kontakt stehend eingesetzt. Hierbei ist es bevorzugt, dass die Oxidationskatalysatoren in diesen Träger y. eingearbeitet sind. Als geeignete Träger y. kommen grundsätzlich die im Zusammenhang mit den Trägern x. genannten Verbindungen in Betracht, wobei Träger auf Siliciumoxid - oder Aluminiumoxid- oder Aluminiumsiliciumoxid-Basis besonders bevorzugt sind. Derartige Oxidationskatalysatoren sind in der Literatur eingehend beschrieben. Hierzu wird beispielhaft auf DE-A-26 26 887, EP-A-0 534 294 sowie auf US-A-2002/0198406 verwiesen. Derartige Oxidationskatalysatoren für die Umsetzung von Acrolein zu Acrylsäure sind beispielsweise von der Mitsubishi Corp., Japan kommerziell erhältlich.

Weiterhin ist es in dem erfindungsgemäßen Verfahren bevorzugt, dass das Dehydratisierungsprodukt in einer wässrigen Phase der Gasphasenoxidation zugeführt wird. Hierbei ist es bevorzugt, dass das Dehydratisierungsprodukt mindestens 10 Gew.-%, bevorzugt mindestens 20 Gew.-% und darüber hinaus bevorzugt mindestens 40 Gew.-% Acrolein aufweist. Die Wassermenge sollte in einem Bereich von 0,1 bis 50 Gew.-%, vorzugsweise in einem Bereich von 10 bis 40 Gew.-% und darüber hinaus bevorzugt in einem Bereich von 12 bis 20 Gew.-% liegen, wobei sich diese und die vorstehenden Gew.-%-Angaben jeweils auf die in die Gasphasenoxidation eingespeiste Phase beziehen.

Die Gasphasenoxidation wird bevorzugt in einem Temperaturbereich von 200 bis 400°C, vorzugsweise in einem Bereich von 250 bis 350°C und darüber hinaus bevorzugt in einem Bereich von 280 bis 340°C durchgeführt.

Ferner ist es in dem erfindungsgemäßen Verfahren bevorzugt, dass das Monomergas die Acrylsäure in einer Menge in einem Bereich von 5 bis 50 Gew.-%, vorzugsweise in einem Bereich von 10 bis 40 Gew.-% und darüber hinaus bevorzugt in einem Bereich von 15 bis 30 Gew.-%, jeweils bezogen auf das Monomergas, beinhaltet.

In einer weiteren Ausgestaltung des erfindungsgemäßen Verfahrens ist es bevorzugt, als Quenchmittel im Verfahrensschritt c) des erfindungsgemäßen Verfahrens Wasser oder eine organische Verbindung mit einem Siedepunkt in einem Bereich von 50 bis 250°C, vorzugsweise in einem Bereich von 70 bis 180°C und darüber hinaus bevorzugt in einem Bereich von 105 bis 150°C oder Wasser und diese organische Verbindung einzusetzen. Als derartige organische Verbindung kommen insbesondere Aromaten und darüber hinaus bevorzugt alkylierte Aromaten in Betracht. Üblicherweise wird das Quenchmittel mit dem Monomergas in einer geeigneten Kolonne, vorzugsweise im Gegenstrom, in Kontakt gebracht. Für den Fall, dass das Quenchmittel zu mindestens 50 Gew.-%, vorzugsweise mindestens 70 Gew.-%, aus Wasser besteht, ist es bevorzugt, dass das mit Acrylsäure befrachtete wässrige Quenchmittel in einem weiteren Schritt mit einem Trennmittel, das vorzugsweise nicht mit Wasser gut löslich ist, aufgearbeitet wird. Die acrylsäurereichste Phase wird entweder einer Destillation oder einer Kristallisation oder beidem, vorzugsweise zunächst einer Kristallisation, unterzogen. Die Kristallisation kann sowohl als Schicht- als auch als Suspensionskristallisation durchgeführt werden. Geeignete Schichtkristallisationsvorrichtungen sind kommerziell von der Sulzer AG zu erhalten. Geeignete Suspensionskristallisationsverfahren bedienen sich in der Regel eines Kristallerzeugers gefolgt von einer Waschkolonne. Derartige Vorrichtungen und Verfahren sind von der Niro Prozesstechnologie BV kommerziell zu erhalten. Als Extraktions/Trennmittel kommt insbesondere eine aromatische Verbindung, darüber hinaus bevorzugt ein Alkylaromat und weiterhin bevorzugt Toluol in Betracht. Sollte eine organische Verbindung als Trennmittel eingesetzt werden, so kann diese mit Acrylsäure befrachtete organische Verbindung ebenfalls sowohl einer Destillation als auch einer Kristallisation oder einer Kombination von beiden unterzogen werden. Eine hierfür geeignete Kristallisation ist in EP-A-1 05 410 offenbart.

Zudem ist es in dem erfindungsgemäßen Verfahren bevorzugt, dass die Quenchphase die Acrylsäure in einer Menge in einem Bereich von 30 bis 90 Gew.-%, vorzugsweise in einem Bereich von 35 bis 85 Gew.-%, und darüber hinaus bevorzugt in einem Bereich von 45 bis 75 Gew.-%, jeweils bezogen auf die Monomerphase, beinhaltet.

In einer weiteren Ausgestaltung des erfindungsgemäßen Verfahrens ist es bevorzugt, dass die Aufarbeitung der Quenchphase bei Temperaturen unterhalb des Siedepunktes des Acrylsäure erfolgt. Eine dafür geeignete Maßnahme ist es, dass die Quenchphase durch Verwendung eines entsprechend kalten Quenchmittels bereits eine Temperatur von unter 40°C aufweist. Die so temperierte Quenchphase kann dann einer Extraktion oder Kristallisation oder beides zur Aufarbeitung zugeführt werden, wobei die Temperaturen vorzugsweise in einem Bereich von -40 bis 40°C, vorzugsweise in einem Bereich von -20 bis 39°C und besonders bevorzugt in einem Bereich von -10 bis 35°C liegen.

Nach einer weiteren Ausgestaltung des erfindungsgemäßen Verfahrens ist es bevorzugt, dass die Monomerphase die Acrylsäure in einer Menge in einem Bereich von 99 bis 99,98 Gew.-%, jeweils bezogen auf die Monomerphase, beinhaltet. Derartige Acrylsäurengehalte in einer Monomerphase treten insbesondere auf, wenn die Aufarbeitung auf destillativem Wege erfolgt. Für den Fall, dass die Aufarbeitung auf dem Weg Extraktion und Kristallisation erfolgt, kann es bevorzugt sein, dass die Acrylsäure in einer Menge von 30 bis 70 Gew.-%, vorzugsweise in einer Menge in einem Bereich von 40 bis 60 Gew.-% und darüber hinaus bevorzugt in einer Menge in einem Bereich von 45 bis 65 Gew.-% in der Monomerphase neben Wasser vorliegen und die von Wasser und Acrylsäure verschiedenen Verunreinigungen weniger als 0,02 Gew.-%, bezogen auf die Monomerphase, betragen. Diese wässrige Monomerphase hat den Vorteil, dass diese ohne weitere Verdünnungsschritte, die bei der hochkonzentrierten Monomerphase notwendig ist, in die wässrige Polymerisation der Monomerphase eingesetzt werden kann.

Nach einer anderen Ausgestaltung des erfindungsgemäßen Verfahrens wird die Menge eines die Vielzahl der Gasblasen erzeugenden Gases variiert. Diese Variation erfolgt über die Zeit. So ist es bevorzugt, dass die Gasmenge mindestens 1-mal pro Sekunde überprüft wird und je nach Bedürfnis des erfindungsgemäßen Verfahren um mindestens 1 Vol.-% erhöht oder erniedrigt wird. Bei gleichmäßigem Verfahrensverlauf können die Variationsintervalle auch länger, beispielsweise im 1 bis 100-minütigen Intervallen, erfolgen. Durch die Variation der Gasmenge lassen sich die Ausbeute beispielsweise über die Gasblasendicht und - Größe steuern. Weiterhin kann durch diese Variation flexibel auf die Zusammensetzung, Art und Qualität der eingesetzten Edukte reagiert werden. Weiterhin kann durch die Variation der Gasmenge auch die Konzentration des in dem Gasphasenoxidationsreaktor weiter umzusetzenden Acroleins gesteuert werden, so dass auch hier optimale Reaktionsbedingungen eingestellt werden.

Es entspricht einer weiteren Ausgestaltung des erfindungsgemäßen Verfahrens, dass das Glycerin vor der Dehydratisierung erwärmt wird. Dieses kann vorzugsweise dadurch erfolgen, indem das Glycerin vor dem Eintritt in den Dehydratisierungsreaktor erwärmt wird. Diese Erwärmung erfolgt vorzugsweise so, dass das vorgewärmte Glycerin in den Dehydratisierungsreaktor eingespeist wird. Es ist bevorzugt, dass das Glycerin auf eine Temperatur in einem Bereich von 150 bis 350°C, bevorzugt in einem Bereich von 250 bis 310°C und besonders bevorzugt in einem Bereich von 270 bis 290°C erwärmt wird. Nicht nur im Fall des Vorwärmens des Glycerins, sondern auch allgemein im Zusammenhang mit dem erfindungsgemäßen Verfahren ist es bevorzugt, dass ein flüssiger Dehydratisierungskatalysator, vorzugsweise Phosphorsäure oder Schwefelsäure eingesetzt wird. Weiterhin ist es in einer anderen Ausgestaltung des erfindungsgemäßen Verfahrens bevorzugt, dass der flüssige Dehydratisierungskatalysator vor der Dehydratisierung erwärmt wird. Dieses kann zusammen mit dem Glycerin erfolgen, es ist jedoch bevorzugt, dass der flüssige Dehydratisierungskatalysator getrennt von dem Glycerin erwärmt wird. Der flüssige Dehydratisierungskatalysator wird vorzugsweise auf eine Temperatur in einem Bereich von 150 bis 350°C, bevorzugt in einem Bereich von 250 bis 310°C und besonders bevorzugt in einem Bereich von 270 bis 290°C erwärmt.

Zudem betrifft die Erfindung eine Vorrichtung zur Herstellung von Acrylsäure, die fluidleitend miteinander verbunden folgende Komponenten aufweist:

    1a. einen Dehydratisierungsreaktor;
    2a. einen Gasphasenoxidationsreaktor;
    3a. eine Quencheinheit;
    4a. eine Aufarbeitungseinheit,

wobei der Dehydratisierungsreaktor einen Gasblasenerzeuger beinhaltet, und wobei mindestens innerhalb eines Teilbereichs des Dehydratisierungsreaktors (2) mindestens ein durchströmbarer Einbau (24) vorgesehen ist.

Zudem betrifft die Erfindung eine Vorrichtung zur Herstellung von Polymeren, die fluidleitend miteinander verbunden zunächst die vorstehend aufgeführten Komponenten 1a. bis 4a. und darüber hinaus eine Polymerisationseinheit 5b. aufweist.

Unter fluidleitend wird eine Verbindung der einzelnen Komponenten oder ihrer Bestandteile durch Rohrleitungssysteme oder andere Transportmöglichkeiten für Gase und Flüssigkeiten, wie Tankwagen, verstanden.

Bei der erfindungsgemäßen Vorrichtung ist es bevorzugt, dass der Dehydratisierungsreaktor einen zur Aufnahme von Glycerin geeigneten Eduktbehälter, gefolgt von einem einen zur Aufnahme von Katalysator ausgestalteten Reaktionsbereich, wiederum gefolgt von einem als Wärmeaustauscher ausgebildeten Quencher mit einem Auslass zu dem Gasphasenoxidationsreaktor aufweist, wobei zwischen dem Auslass und dem Gasphasenoxidationsreaktor noch ein Abscheider zum Trennen von gasförmigen und flüssigen Komponenten sowie ggf. zur Aufreinigung der in dem Abscheider anfallenden flüssigen Phase eine Destillationskolonne als Trenneinheit vorgesehen sein. Diese Komponenten sind aus üblichen, für die chemische Industrie verwendeten, im Zusammenhang mit den Reaktionsbedingungen inerten Materialien, wie Edelstahl oder Glas ausgebildet. In der unteren Hälfte des Reaktors sind ein oder mindestens zwei Gasblasenerzeuger vorgesehen, die vorzugsweise als Fritte ausgestaltet ist, die meist aus Metall besteht. Für den Fall, dass der Reaktionsbereich den Katalysator als Schüttgut aufnimmt, weist dieser entsprechende Behälter auf. In einer anderen Ausgestaltung kann der Reaktionsbereich auch Wandungen aufweisen, die als Katalysator fungieren.

Sollte neben oder anstelle des Feststoffkatalysators ein Flüssigkatalysator eingesetzt werden, ist es bevorzugt, auch diesen in einem Tank zu bevorraten. An den Dehydratisierungsreaktor schließt sich in einer bevorzugten Ausgestaltung des erfindungsgemäßen Verfahrens ein Wärmetauscher an, in dem das aus dem Dehydratisierungsreaktor stammende Gas abgekühlt wird. Eine weitere Ausgestaltung sieht vor, dass auf den Dehydratisierungsreaktor und vor dem Gasphasenoxidationsreaktor ein Phasentrennbehälter mittelbar oder unmittelbar folgt, in dem als eine Flüssigphase mit mehr Acrolein als in der gleichfalls dort erhaltenen Gasphase anfällt. Diese acroleinarme Gasphase kann zur Regulierung der Gasmengen und damit der Konzentrationsverhältnisse in den Gasphasenoxidationsreaktor eingespeist werden. Ferner entspricht es einer Ausgestaltung, dass zwischen Dehydratisierungs- und Gasphasenoxidationsreaktor eine thermische Trenneinheit, die vorzugsweise als Destillationskolonne ausgestaltet ist, angeordnet ist. In dieser thermischen Trenneinheit wird Acrolein als Leichtsieder von Schwersiedern und auch Glycerin abgetrennt, wobei dass Glycerin wieder dem Dehydratisierungsreaktor zugeführt wird. Das so erhaltene, aufgereinigte als Gasphase vorliegende Acrolein wird, ggf. noch weitere gasförmige Bestandteile beisichführend, dann dem Gasphasenoxidaitonsreaktor zugeführt. In einer anderen Ausgestaltung werden stromab des Dehydratisierungsreaktors der Wärmetauscher und der Phasentrennbehälter und thermische Trenneinheit vor dem Gasphasenoxidationsreaktor angeordnet. In einer anderen Ausgestaltung der erfindungsgemäßen Vorrichtung weist diese eine Inertgasführung auf, mit der zum einem das Gas zur Erzeugung der Gasblasen in dem Dehydratisierungsreaktor und zum anderen Gas in den Gasphasenoxidationsreaktor eingespeist wird.

Mindestens innerhalb eines Teilbereichs des Dehydratisierungsreaktors ist ein durchströmbarer Einbau vorgesehen. Es ist weiterhin bevorzugt, dass der durchstömbare Einbau mindestens in einem Teilbereich stern-, kreuz-, platten-, kugel-, schlaufen-, ring- oder rohrförmig oder in mindestens zwei dieser Formen ausgestaltet ist, wobei platten- und rohrförmige bevorzugt und rohrförmige besonders bevorzugt sind. Im Zusammenhang mit dem durchströmbaren Einbau wird zunächst auf die hier erfolgten Ausführungen zu der Leitung, Teilung und Bremsung der Gasblasen Bezug genommen.

Weiterhin kommen als durchströmbare Einbauten alle dem Fachmann bekannten und geeignet erscheinenden Ausgestaltungen wie Platten, Waben, Ringe, Geflechte, Rohre oder Kombinationen daraus grundsätzlich in Betracht. Die Einbauten können sowohl als Einsätze, als auch fest verbunden oder einstückig mit dem Dehydratisierungsreaktor ausgebildet sein. Ferner können die erfindungsgemäß geeigneten durchströmbaren Einbauten auch aus der Reaktorwand des Dehydratisierungsreaktors heraus ausgebildet sein. Dieses kann beispielsweise durch in den Innenraum des Dehydratisierungsreaktors ragende Ausstülpungen der Reaktorwand bewerkstelligt werden. Durch die Ausgestaltung und Dimensionierung der Durchströmungsräume der durchströmbaren Einbauten kann sowohl die Größe als auch die Durchströmungsgeschwindigkeit der Gasblasen in der Flüssigphase a1 kontrolliert werden. So gilt allgemein, dass die Größe der Gasblasen durch den jeweiligen Strömungsquerschnitt der durchströmten Räume der durchströmbaren Einbauten festgelegt wird. Dieser bestimmt sich dadurch, dass in einem dem Querschnitt des Dehydratisierungsreaktors entsprechenden Einbautequerschnitt ein Kreis, der dem Querschnitt der Gasblasen am nächsten kommt, so gelegt wird, dass dieser Kreis mindestens an drei Stellen des Einbautenquerschnitts tangential anliegt und der Strömungsquerschnitt das doppelte des Radius dieses Kreises ist. Allgemein wird der Strömungsquerschnitt der Einbauten mindestens so groß wie der einzustellende Gasblasendurchmesser gewählt. Hiernach ist es bevorzugt, dass mindestens in einem Bereich des durchströmten Raums der Strömungsquerschnitt in einem Bereich von 0,1 bis 100 mm, vorzugsweise von 1 bis 70 mm und darüber hinaus bevorzug in einem Bereich von 5 bis 40 mm liegt.

Weiterhin kann die Wanderungsgeschwindigkeit und damit die Verweilzeit der Gasblasen und der Gasmenge in der Flüssigphase a1 auch durch die Oberflächenbeschaffenheit der durchströmbaren Bauten, insbesondere sofern diese im Kontakt mit den Gasblasen und der Flüssigphase a1 steht, beeinflusst werden. Hierbei eignen sich insbesondere raue Oberflächen, wobei die Rauigkeit der Oberflächen nach der Zusammensetzung der Flüssigphase a1 und der in den Gasblasen enthaltenen Gase richtet.

Durchströmbare Einbauten sind vorzugsweise Trennbleche, insbesondere parallel, kreuz-, strich-, oder sternförmig angeordnet, zwei oder mehrere, vorzugsweise bündelartig im Reaktor angeordnete Röhren, Geflechte, Gewirke, Ringe, Ketten, Kegel, insbesondere Hohlkegel, oder Bürsten oder eine Kombination aus zwei oder mehreren der vorstehend aufgelisteten möglichen Einbautenvarianten. Die durchströmbaren Einbauten können auch grundsätzlich allen, dem Fachmann hierfür bekannten und geeignet erscheinenden Materialien gebildet sein. Als Materialien sind insbesondere Keramik, Glas und Stahl bevorzugt. Wichtig bei der Auswahl des geeigneten Materials ist, dass dieses möglichst beständig gegenüber den, während der Dehydratisierungsreaktoin, herrschenden Bedingungen ist. Hiernach besonders geeignet sind Stahlrohrbündel, Geflechte, wie beispielsweise von der Cal Gavin Ltd. angebotenen bürstenartig ausgestalteten länglichen Drahtgeflechte, Keramik und/oder Glasringe, wie sie beispielsweise in Destillationskolonnen eingesetzt und als Raschigringe bekannt sind.

Einer weiteren erfindungsgemäßen Ausgestaltung entsprechend, nehmen die durchströmbaren Einbauten nur einen Teil des Dehydratisierungsreaktors ein. Hierbei handelt es sich vorzugsweise um den Teil, in dem auch währen der Reaktion die Flüssigphase a1 sich befindet. Außerdem ist es bevorzugt, dass die durchströmbaren Einbauten zu dem Gasblasenerzeuger beabstandet vorgesehen sind. Bezogen auf die Mittelachse des Dehydratisierungsreaktors in Längsrichtung ist es bevorzugt, dass die durchströmbaren Einbauten zwischen 5 und 95 %, vorzugsweise zwischen 50 und 90 % und besonders bevorzugt zwischen 70 und 85% der auf dies Achse bezogenen Gesamtlänge des Dehydratisierungsreaktors einnehmen. Weiterhin ist es bevorzugt, dass, bezogen auf die in Strömungsrichtung betrachtete Längesachse, im Einströmungs- sowie im Ausströmungsbereich des Dehydratisierungsreaktors weniger durchströmbare Einbauten vorgesehen sind, als in den zwischen dem Ein- und Ausströmungsbereich befindlichen Reaktionsbereich.

In einer weiteren Ausgestaltung der erfindungsgemäßen Vorrichtung ist es bevorzugt, dass der Dehydratisierungsreaktor einen sich zu einem Auslass verjüngenden oberen Bereich aufweist. Diese Verjüngung kann sowohl gradlinig als auch gekrümmt oder in einer Kombination aus gradlinig und gekrümmt erfolgen. Im Fall der gekrümmten Verjüngung kann diese Krümmung konkav oder konvex sein. Oftmals kann die Verjüngung kornisch als auch teilkugelförmig ausgebildet sein. Im Fall der kornischen Ausgestaltung ist die Verjüngung im Wesentlichen stumpfkegelförmig. Grundsätzlich gestaltet der Fachmann die Verjüngung so, dass die aus der unterhalb der Verjüngung beim Betrieb des Reaktors liegende Flüssigphase austretenden Gase beim Durchströmen der Verjüngung beschleunigt werden. Es weiterhin möglich, dass neben den Gasen auch die über die Verjüngung austretenden Flüssigkeiten beschleunigt werden. In einer weiteren Ausgestaltung ist es bevorzugt, dass au die Verjüngung wieder eine Erweiterung erfolgt, bei der die durchströmenden Gase entschleunigt werden.

Weiterhin ist es in einer Ausgestaltung der erfindungsgemäßen Vorrichtung bevorzugt, dass dem Dehydratisierungsreaktor mindestens ein Wärmetauscher vorgelagert ist. Hierbei ist bevorzugt, dass der oder die Wärmetauscher so nah an den Dehydratisierungsreaktor angeordnet sind, dass es zu keiner nennenswerten Abkühlung zwischen Wärmetauscher und Dehydratisierungsreaktor kommen kann. Vorzugsweise ist mindestens ein Wärmetauscher für das Glycerin als Edukt und für den flüssigen Katalysator vorgesehen. Für den Fall, dass kein Flüssigkatalysator eingesetzt wird, ist es ausreichend, für das Glycerin einen Wärmetauscher in der erfindungsgemäßen Vorrichtung vorzusehen.

Eine Weiterbildung der erfindungsgemäßen Vorrichtung weist nach dem Eduktbehälter und vor dem Reaktionsbereich einen Verdampfer auf. Diese Ausgestaltungen eignen sich insbesondere für die Gasphasendehydratisierung. Für den Fall, dass das Glycerin aus der Fettsäureverseifung mit einer hohen Salzfracht eingesetzt wird, ist es bevorzugt, dass der Verdampfer einen Salzabscheider aufweist.

Als Gasblasenerzeuger kann allgemein jede dem Fachmann dazu geeignet erscheinende Vorrichtung eingesetzt werden. Es ist bevorzugt, dass der Gasblasenerzeuger in der unteren Hälfte des Reaktors angeordnet ist, so dass eine möglichst vorteilhafte und vollständige Durchströmung der Flüssigphase in dem Dehydratisierungsreaktor erreicht werden kann. Geigente Glasblasenerzeuger sind beispielsweise Fritten aus Metall oder Glas aus denen die Gasblasen herausperlen, Injektoren, die gegen eine Prallplatte gerichtet sein können, oder nach dem Venturi-Prinzip arbeitende Injektoren. Die Injektoren könne auch mit einem statischen Mischer kombiniert werden, der den Gasstrom aus dem Injektor in kleine Gasblasen zerteilt und diese möglichst homogen in dem Reaktor verteilt.

Als Gasphasenoxidationsreaktoren kommen alle dem Fachmann geläufigen und für das erfindungsgemäße Verfahren geeignet erscheinenden Reaktoren in Betracht, die in der Lage sind, Acrolein durch Gasphasenoxidation zu Acrylsäure umzusetzen. In diesem Zusammenhang bevorzugt sind Rohrbündelreaktoren oder Plattenreaktoren, die mit einem Kühlmittel, vorzugsweise einer Salzschmelze, gekühlt werden. Diese Rohrbündel- oder Plattenreaktoren nehmen auf der dem Kühlmittel abgewandten Seite einen geeigneten Katalysator auf. Dieser kann zum einen als Pulverschüttung vorliegen und zum anderen können die Flächen der Rohre bzw. der Platten mit dem Katalysator beschichtet sein.

Als Quencheinheiten werden ebenso die bei der bisherigen großtechnischen Gasphasenoxidation von Acrolein zu Acrylsäure gängigen Typen bevorzugt eingesetzt. Derartige Quencheinheiten sind als Kolonnen oder Türme ausgebildet und können genauso wie die Reaktoren beispielsweise von der Deggendorfer Werft GmbH kommerziell erworben werden. Als Aufarbeitungseinheit kommen ebenso alle dem Fachmann aus der großtechnischen über Gasphasenoxidation von Acrolein laufenden Acrylsäuresynthese bekannten Destillations- und Kristallisationssowie Extraktionseinrichtungen in Betracht.

Als Polymerisationseinheiten, welche im Verfahrensschritt E. zur Polymerisation der Monomerphase eingesetzt wird, sind zum einen diskontinuierlich arbeitende Rührkessel und zum anderen kontinuierlich arbeitende Systeme, wie Bandpolymerisationsvorrichtungen, Extruder und dergleichen geeignet. Auf diese Polymerisationsreaktoren folgt eine Zerkleinerung und Trocknung. Der so erhaltene Superabsorberprecursor kann weiterhin einer Oberflächen- oder Nachvernetzung unterzogen werden. Hierzu finden sich nähere Angaben in dem eingangs erwähnten Werk von Graham & Buchholz. Wenn es sich bei den Polymeren um vernetzte, teilneutralisierte Polyacrylate handelt, so wird hinsichtlich der genauen Vorgehensweise auf das 3. Kapitel (Seite 69ff) in "Modern Superabsorbent Polymer Technology", F. L. Buchholz und A. T. Graham (Herausgeber) in, Wiley-VCH, New York, 1998 verwiesen, das einen Teil dieser Offenbarung bildet.

Zudem ist es bevorzugt, dass das erfindungsgemäße Verfahren zur Herstellung von Acrylsäure bzw. das erfindungsgemäße Verfahren zur Herstellung eines Polymers unter Einsatz der vorstehend beschriebenen und in den Zeichnungen näher erläuterten Vorrichtungen erfolgt.

Auf diese Art und Weise lassen sich wasserabsorbierende Polymergebilde als besonders geeignete Superabsorber erhalten.

Einen Beitrag zur Lösung der eingangs genannten Aufgaben leisten auch wasserabsorbierende Polymergebilde, erhältlich durch radikalische Polymerisation der durch das vorstehend beschriebene Syntheseverfahren erhältlichen Acrylsäure in Gegenwart von Vernetzern.

Einen Beitrag zur Lösung der eingangs genannten Aufgaben leisten auch wasserabsorbierende Polymergebilde, welche zu mindestens 25 Gew.-%, vorzugsweise zu mindestens 50 Gew.-%, noch mehr bevorzugt zu mindestens 75 Gew.-% und am meisten bevorzugt zu mindestens 95 Gew.-%, auf Acrylsäure basieren, wobei die wasserabsorbierenden Polymergebilde durch einen Nachhaltigkeitsfaktor von mindestens 80 % gekennzeichnet sind.

Der Nachhaltigkeitsfaktor gibt an, zu welchem Anteil das Polymergebilde auf Stoffen basierend auf nichtfossilen, nachwachsendem organischen Material basiert. Bei einem Nachhaltigkeitsfaktor von 100 besteht das Polymergebilde vollständig aus auf nichtfossilen, nachwachsendem organischen Materialen basierenden Stoffen.

Einer anderen Ausgestaltung entspricht ein wasserabsorbierende Polymergebilde, welches zu mindestens 25 Gew.-%, vorzugsweise zu mindestens 50 Gew.-%, noch mehr bevorzugt zu mindestens 75 Gew.-% und am meisten bevorzugt zu mindestens 95 Gew.-%, auf Acrylsäure basiert, wobei mindestens 80 Gew.-%, vorzugsweise mindestens 90 Gew.-% und am meisten bevorzugt mindestens 95 Gew.-% der zur Herstellung der wasserabsorbierenden Polymergebilde eingesetzten Acrylsäuremonomere durch ein Syntheseverfahren erhalten wurden, welches von nichtfossilem, nachwachsendem organischen Material ausgeht. Bei diesen nichtfossilen, nachwachsenden organischen Materialien handelt es sich insbesondere nicht um aus Erdöl, oder Stein- bzw. Braunkohle sowie Erdgas gewonnnen Materialien. Vielmehr sind diese nichtfossilen, nachwachsenden organischen Materialien Erzeugnisse der Land- und Forstwirtschaft, insbesondere Fette und Öle aus Glycerin und Fettsäuren.

Vorzugsweise sind diese wasserabsorbierende Polymergebilde erhältlich durch ein Verfahren umfassend die folgenden Verfahrensschritte:

> i) Polymerisation der Acrylsäure in Gegenwart eines Vernetzers unter Ausbildung eines Polymergels;
> ii) gegebenenfalls Zerkleinerung des Polymergels;
> iii) Trocknung des gegebenenfalls zerkleinerten Polymergels unter Erhalt wasserabsorbierender Polymergebilde, sowie
> iv) gegebenenfalls Oberflächennachbehandlung der wasserabsorbierenden Polymergebilde.

Gemäß einer besonderen Ausführungsform der wasserabsorbierende Polymergebilde basieren diese zu mindestens 20 Gew.-%, vorzugsweise zu mindestens 35 Gew.-% und am meisten bevorzugt zu mindestens 45 Gew.-% auf natürlichen, biologisch abbaubaren Polymeren, vorzugsweise auf Kohlehydraten wie etwa Zellulose oder Stärke.

Im Zusammenhang mit dem Wasserabsorbierendes Polymergebilde ist es bevorzugt, dass dieses die folgenden Eigenschaften aufweist:

> $\underline{A}$ einen gemäß der hierin beschriebenen Testmethode bestimmte Saline Flow Conductivity (SFC) von mehr als 30 x $10^{-7}$ cm$^3$s/g, vorzugsweise von mehr als $60 \times 10^{-7}$ cm$^3$s/g, besonders bevorzugt von mehr als 90 x $10^{-7}$ cm$^3$s/g und ferner bevorzugt von mehr als 120 x $10^{-7}$ cm$^3$s/g sowie weiterhin bevorzugt von mehr als 130 x $10^{-7}$ cm$^3$s/g und am meisten bevorzugt von mehr als 140 x $10^{-7}$ cm$^3$s/g;
> $\underline{B}$ eine nach ERT 442.2-02 bestimmte Absorption gegen einen Druck von 0,7 psi (AAP$_{0.7}$) von mehr als 15 g/g, vorzugsweise von mehr als 16 g/g, bevorzugt von mehr als 17 g/g, besonders bevorzugt von mehr als 19 g/g und ferner bevorzugt von mehr als 20 g/g sowie am meisten bevorzugt von mehr als 22 g/g;
> $\underline{C}$ eine nach ERT 441.2-02 bestimmte Retention (CRC) von mehr als 20 g/g, vorzugsweise von mehr als 21 g/g, bevorzugt von mehr als 22 g/g, besonders bevorzugt von mehr als 23 g/g und ferner bevorzugt von mehr als 25 g/g sowie am meisten bevorzugt von mehr als 27 g/g.

Es ist möglich, die Eigenschaften wie SFC, AAP und CRC auch nach oben hin zu begrenzen. Derartige Obergrenzen liegen für den SFC in einigen Fällen bei 180 x $10^{-7}$ cm$^3$s/g oder erst bei $200 \times 10^{-7}$ cm$^3$s/g und manchmal auch bei $250 \times 10^{-7}$ cm$^3$s/g oder bei $350 \times 10^{-7}$ cm$^3$s/g oder auch bei 500x $10^{-7}$ cm$^3$s/g. Obergrenzen für den AAP liegen bei 30 g/g in manchen Fällen bei 35 g/g und gelegentlich bei 45 g/g. Obergrenzen für den CRC liegen bei 35 g/g in manchen Fällen bei 45 g/g und gelegentlich bei 50 g/g.

Es ist weiterhin bevorzugt, dass das wasserabsorbierende Polymergebilde zusätzlich zu den Eigenschaften $\underline{A}$ bis $\underline{C}$ noch folgendes aufweist:

> $\underline{D}$ eine gemäß dem modifizierten Sturm-Test gemäß Anhang V zur Richtlinie 67/548/EWG bestimmte biologische Abbaubarkeit nach 28 Tagen von mindestens 25 %, vorzugsweise mindestens 35 % und am meisten bevorzugt mindestens 45 % auf, wobei ein Wert von maximal 75 bis 95 % als Obergrenze im Allgemeinen nicht überschritten wird.

Weiterhin ist es bevorzugt, dass das wasserabsorbierende Polymergebilde eine Vielzahl anorganischer Feinteilchen beinhaltet. Als anorganische Feinteilchen können alle wasserunlöslichen, anorganischen Verbindungen eingesetzt werden, aus denen stabile, kolloiddisperse, vorzugsweise einphasige, wässrige Lösungen erhalten werden können, die bei 20°C und Normaldruck über einen Zeitraum von mindestens 6h, bevorzugt mindestens 24h und besonders bevorzugt mindestens 72h bis hin zu 6 Monaten keine Phasentrennung, wie etwa das Absetzen eines festen, anorganischen Niederschlags, zeigen.

Unter einer kolloiddispersen Lösung wird vorzugsweise eine Lösung verstanden, die Partikel mit einem Partikeldurchmesser in einem Bereich von 100-1000 Å (10-4 bis 10-5 cm) enthält. Diese Lösungen besitzen die Eigenschaft, einen durch die Lösung geschickten Lichtstrahl in alle Richtungen zu streuen, so dass der Gang des Lichtstrahls durch die kolloiddisperse Lösung verfolgt werden kann (Tyndall Effekt, siehe hierzu Hollemann·Wiberg, Lehrbuch der anorganischen Chemie, 91.-100. Auflage, de Gruyter-Verlag, Seite 765).

Im Zusammenhang mit den wasserabsorbierenden Polymergebilde ist es bevorzugt, dass die anorganischen Feinteilchen Sauerstoff beinhalten. Ferner ist es bevorzugt die anorganischen Feinteilchen ein Metall beinhalten. Als besonders bevorzugte kolloiddisperse anorganische Verbindung werden im erfindungsgemäßen Verfahren Polykieselsäure beinhaltende Partikel eingesetzt. Eine kolloiddisperse Lösung enthaltend solche Partikel (Kieselsäuresol) kann beispielsweise durch vorsichtiges Ansäuern von infolge Hydrolyse alkalische reagierenden Natriumsilicatlösungen erhalten werden oder aber durch Lösen molekularer Kieselsäure in Wasser und eventueller anschließender Stabilisierung der entstehenden kolloiddispersen Lösung. Die genaue Herstellung derartiger Kieselsäuresole ist dem Fachmann bekannt und ist beispielsweise in Jander·Blasius, "Lehrbuch der analytischen und präparativen anorganischen Chemie" S. Hirzel Verlag, Stuttgart, beschrieben. Neben der kolloiddispersen Kieselsäure sind erfindungsgemäß des weiteren Eisen(III)oxid-Hydrat-Sole, Zinn(IV)oxid-Hydrat-Sole oder auf Silberhalogeniden, insbesondere Silberchlorid, basierende Sole als kolloiddisperse anorganische Verbindung besonders bevorzugt.

Die wasserabsorbierenden Polymergebilde weisen vorzugsweise einen nachvernetzten Außenbereich auf. Meist zeigen derartige wasserabsorbierende Polymergebilde eine Kern-Schale-Morfologie. Vorzugsweise sind die anorganischen Feinteilchen auf oder in oder auf und in dem Außenbereich vorgesehen.

Bevorzugte Polymergebilde sind Fasern, Schäume oder Teilchen, wobei Fasern und Teilchen bevorzugt und Teilchen besonders bevorzugt sind.

Bevorzugte Polymerfasern sind so dimensioniert, dass sie in oder als Garne für Textilien und auch direkt in Textilien eingearbeitet werden können. Es ist erfindungsgemäß bevorzugt, dass die Polymerfasern eine Länge in einem Bereich von 1 bis 500, bevorzugt 2 bis 500 und besonders bevorzugt 5 bis 100 mm und einen Durchmesser in einem Bereich von 1 bis 200, bevorzugt 3 bis 100 und besonders bevorzugt 5 bis 60 Denier besitzen.

Bevorzugte Polymerteilchen sind so dimensioniert, dass sie eine mittlere Teilchengröße gemäß ERT 420.2-02 in einem Bereich von 10 bis 3000 $\mu$m, vorzugsweise 20 bis 2000 $\mu$m und besonders bevorzugt 150 bis 850 $\mu$m aufweisen. Weiterhin ist es bevorzugt, dass der Anteil an Partikeln mit einer Partikelgröße in einem Bereich von 300 bis 600 $\mu$m mindestens 50 Gew.-%, besonders bevorzugt mindestens 75 Gew.-% beträgt.

Einen weiteren Beitrag zur Lösung der eingangs beschriebenen Aufgaben liefert ein Verbund, beinhaltend die wasserabsorbierenden Polymergebilde bzw. wasserabsorbierenden Polymergebilde, die durch radikalische Polymerisation der durch das vorstehend beschriebene Syntheseverfahren erhältlichen Acrylsäure in Gegenwart von Vernetzern erhältlich sind, und ein Substrat. Es ist dabei bevorzugt, dass die erfindungsgemäßen Polymergebilde und das Substrat miteinander fest verbunden sind. Als Substrate sind Folien aus Polymeren, wie beispielsweise aus Polyethylen, Polypropylen oder Polyamid, Metalle, Vliese, Fluff, Tissues, Gewebe, natürliche oder synthetische Fasern, oder andere Schäume bevorzugt. Weiterhin ist es erfindungsgemäß bevorzugt, dass die Polymergebilde in einer Menge von mindestens 50 Gew.-%, vorzugsweise mindestens 70 Gew.-% und noch mehr bevorzugt mindestens 90 Gew.-%, bezogen auf das Gesamtgewicht aus Polymergebilde und Substrat, in dem Verbund enthalten sind.

In einer besonders bevorzugten Ausführungsform des Verbundes handelt es sich um einen flächenförmigen Verbund, wie er in der WO-A-02/056812 als "absorbent material" beschrieben ist. Der Offenbarungsgehalt der WO-A-02/056812, insbesondere hinsichtlich des genauen Aufbaus des Verbundes, des Flächengewichtes seiner Bestandteile sowie seiner Dicke wird hiermit als Referenz eingeführt und stellt einen Teil der Offenbarung der vorliegenden Erfindung dar.

Einen weiteren Beitrag zur Lösung der eingangs genannten Aufgaben liefert ein Verfahren zur Herstellung eines Verbundes, wobei die wasserabsorbierenden Polymergebilde bzw. die wasserabsorbierenden Polymere, die durch radikalische Polymerisation der durch das vorstehend beschriebene Syntheseverfahren erhältlichen Acrylsäure in Gegenwart von Vernetzern erhältlich sind, und ein Substrat und gegebenenfalls ein Zusatzstoff miteinander in Kontakt gebracht werden. Als Substrate werden vorzugsweise diejenigen Substrate eingesetzt, die bereits vorstehend im Zusammenhang mit dem Verbund genannt wurden.

Einen Beitrag zur Lösung der eingangs genannten Aufgaben liefert auch ein Verbund erhältlich nach dem vorstehend beschriebenen Verfahren.

Einen weiteren Beitrag zur Lösung der eingangs genannten Aufgaben liefern chemische Produkte beinhaltend die wasserabsorbierenden Polymergebilde oder einen erfindungsgemäßen Verbund. Bevorzugte chemische Produkte sind insbesondere Schäume, Formkörper, Fasern, Folien, Filme, Kabel, Dichtungsmaterialien, flüssigkeitsaufnehmenden Hygieneartikel, insbesondere Windeln und Damenbinden, Träger für pflanzen- oder pilzwachstumsregulierende Mittel oder Pflanzenschutzwirkstoffe, Zusätze für Baustoffe, Verpackungsmaterialien oder Bodenzusätze. Bevorzugte chemische Produkte sind Hygieneartikel, umfassend eine für Oberschicht, eine für Unterschicht sowie eine zwischen der Oberschicht und der Unterschicht angeordnete Zwischenschicht, welche wasserabsorbierenden Polymergebilde beinhaltet.

Zudem betrifft die Erfindung ein Verfahren zur Herstellung von Acrolein das durch das hierin beschriebene Verfahren zur Dehydratisierung von Glycerin zu einem Acrolein aufweisenden Dehydratisierungsprodukt und die hierin beschriebenen bevorzugten Ausführungsformen dieser Dehydratisierung gekennzeichnet ist.

Beschrieben sind auch Fasern, Folien, Klebstoffe, Kosmetika, Formmassen, Textil- und Lederhilfsmittel, Flockungsmittel, Beschichtungen oder Lacke basierend auf Acrylsäure, die nach einem erfindungsgemäßen Verfahren erhältlich ist, oder deren Derivate oder Salze. Als Derivate der Acrylsäure kommen insbesondere ihre Ester, vorzugsweise ihre Alkylester und darüber hinaus bevorzugt ihre $C_1$- bis $C_{10}$-, darüber hinaus bevorzugt $C_2$- bis $C_5$- und weiterhin bevorzugt $C_3$- bis $C_4$-Alkylester in Betracht. Als Salze sind die Alkali- oder Erdalkali- sowie die Ammoniumsalze der Acrylsäure zu nennen.

Ebenfalls beschrieben ist die Verwendung einer Acrylsäure, die durch ein erfindungsgemäßes Verfahren erhalten wurde, oder deren Derivate oder Salze in Fasern, Folien, Klebstoffen, Kosmetika, Formmassen, Textil- und Lederhilfsmitteln, Flockungsmitteln, Beschichtungen oder Lacken.

Die Erfindung wird nun anhand von Zeichnungen und Beispiele näher erläutert.

Fig. 1    zeigt schematisch einen schematischen Ablauf der einzelnen Stufen und Schritte des erfindungsgemäßen Verfahrens und der erfindungsgemäßen Vorrichtung.

Fig. 2    zeigt schematisch eine Dehydratisierungseinheit gefolgt von einer Gasphasenoxidationseinheit.

Fig. 3    zeigt einen Ausschnitt B aus dem Dehydratisierungreaktor als Längsschnitt.

Fig. 4    zeigt schematisch eine weitere Dehydratisierungseinheit gefolgt von einer Gasphasenoxidationseinheit.

Fig. 5    zeigt schematisch einen Längsschnitt durch einen erfindungsgemäßen Dehydratisierungsreaktor.

Fig. 6    zeigen in a) und b) Ausgestaltungen erfindungsgemäßer durchströmbarer Einbauten im Längsschnitt.

Fig. 7    zeigen in a) bis c) Ausgestaltungen erfindungsgemäßer Einbauten im Querschnitt.

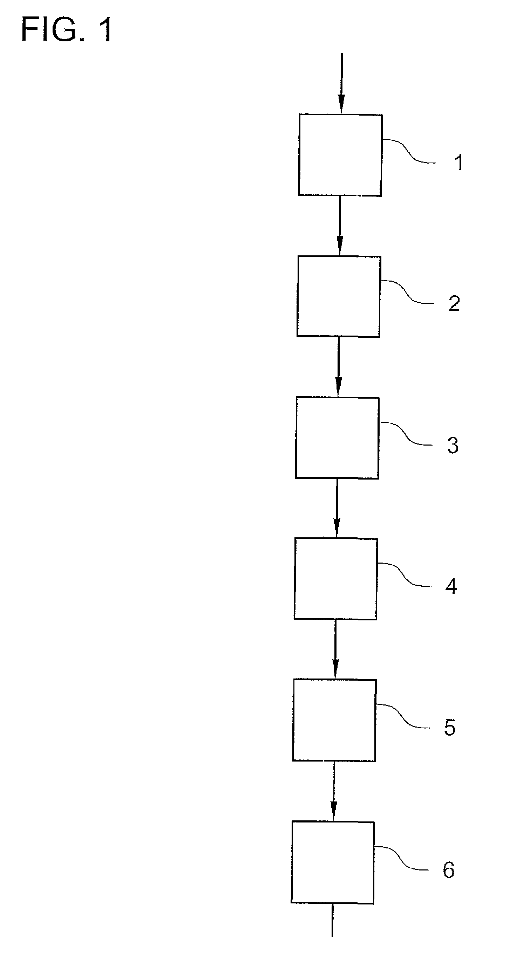

In Fig. 1 werden zunächst die Öle oder Fette in einen Verseifer 1 eingegeben, wo eine alkalische Verseifung mit Laugen oder Alkalialkoholaten erfolgt. Das in dem Verseifer gewonnene Glycerin wird dann einer Dehydratisierungseinheit mit einem Dehydratisierungsreaktor 2 zugeführt (um aus dem Glycerin Acrolein zu gewinnen). Das so gewonnene Acrolein wird dann in einem nächsten Schritt einem Gasphasenreaktor 3 zugeführt, in dem es durch eine Gasphasenoxidationsreaktion zu Acrylsäure umgesetzt wird. Auf den Gasphasenreaktor 3 folgt eine Quencheinheit 4, in der das acrylsäurehaltige Gas aus dem Gasphasenreaktor 3 in die flüssige Phase durch in Kontakt bringen mit einem Quenchmittel gebracht wird. Die flüssige Mischung aus Quenchmittel und Acrylsäure wird einer auf die Quencheinheit 4 folgenden Aufarbeitungseinheit 5 zugeführt. Dort wird die Acrylsäure entweder durch Kristallisation oder Destillation oder einer Kombination dieser beiden Schritte oder auch durch Extraktion oder einer Kombination aus Extraktion und Kristallisation oder einer Kombination aus Extraktion und Destillation oder einer Kombination aus Extraktionsdestillation und Destillation zu reiner Acrylsäure (mindestens 99,98 % Acrylsäure) aufgereinigt, die entweder als reine Acrylsäure für sich oder in einer wässrigen Phase vorliegt. Die so gewonnene Acrylsäure wird dann einer Polymerisationseinheit 6 zugeführt. Das in der Polymerisationseinheit 6 gewonnene Polymer kann entsprechend der nachfolgenden Verwendung konfektioniert werden. Auf die Polymerisationseinheit 6 kann eine Weiterverarbeitungseinheit, beispielsweise eine Windelmaschine oder eine Maschine zur Herstellung von Verbands- und Wundmaterialien folgen.

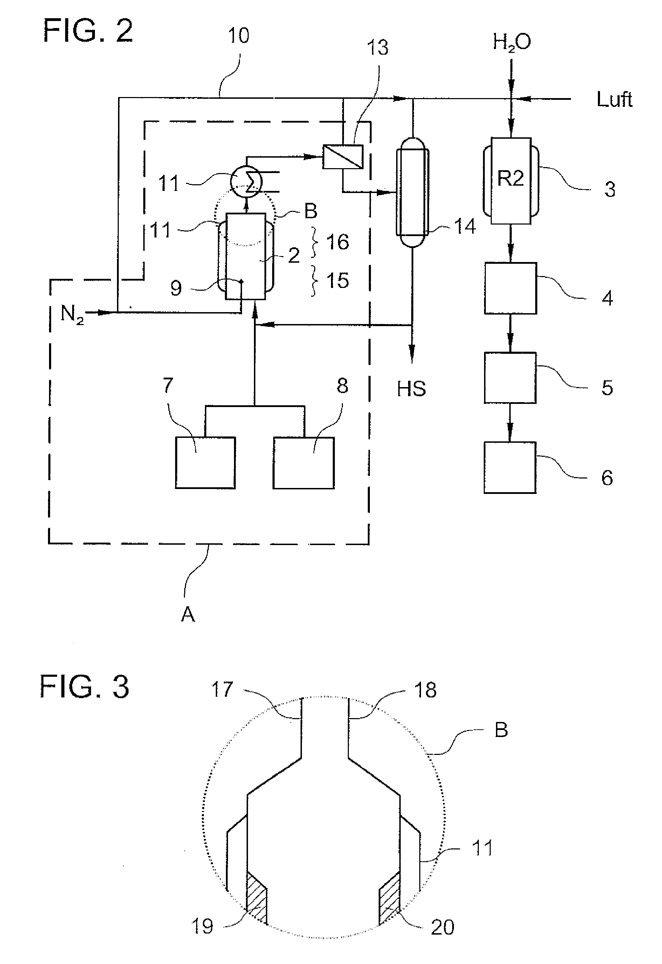

In Fig. 2 wird in einen Dehydratisierungsreaktor 2 aus einem Eduktspeicher 7, der entweder über eine Leitung mit dem Verseifer 1 oder über Transportmittel wie Tankwagen mittelbar mit dem Verseifer 1 verbunden sein kann, eine meist wässrige Glycerinlösung in den unteren Bereich des Dehydratisierungsreaktors 2 eingespeist. Für den Fall, dass Flüssigkatalysator eingesetzt wird, ist ein Flüssigkatalysatorbehälter 8 vorgesehen, der über eine Leitung ebenso mit dem Reaktor 2 verbunden ist, wobei es bevorzugt ist, dass das Glycerin bzw. die wässrige Glycerinphase und der Flüssigkatalysator vor dem Einspeisen in den Reaktor 2 zusammengeführt werden. Dieses kann beispielsweise durch vorheriges Verbinden der beiden Leitungen des Eduktbehälters 7 und des Flüssigkatalysatorbehälters 8 über einen statischen Mischer oder eine andere, dem Fachmann als geeignet erscheinende Mischvorrichtung (nicht gezeigt), erfolgt.

Weiterhin weist der Dehydratisierungsreaktor in seinem unteren Bereich einen als Metallfritte ausgebildeten Gasblasenerzeuger 9, der aus einer Gaszuführung 10 mit zum Erzeugen von Blasen geeigneten Gas versorgt wird. Die Gasführung 10 kann nicht nur mit dem Dehydratisierungsreaktor 2 sondern auch mit dem Gasphasenoxidationsreaktor 3 verbunden sein. Der Dehydratisierungsreaktor 2 ist weiterhin über eine Heizvorrichtung 11 beheizbar. Durch die Heizvorrichtung 11 wird neben dem eingespeisten Gas ein entsprechendes Druckverhältnis in dem Dehydratisierungsreaktor 2 erzeugt, so dass sich zum einen eine flüssige und zum anderen eine gasförmige Phase in dem Dehydratisierungsreaktor 2 ausbildet. Hierbei wird darauf zu achten sein, dass der Gasblasenerzeuger 9 durch die im Unterbereich 15 befindliche flüssige Phase bedeckt ist. An den oberen Bereich des als Druckbehälter ausgestalteten Dehydratisierungsreaktors schließt sich ein Wärmeaustauscher 12 an, in dem das aus dem Oberbereich 16 des Dehydratisierungsreaktors 2 stammende Gas entspannt und abgekühlt wird. An den Wärmetauscher 12 schließt sich ein Abscheidebehälter 13 an in dem die gasförmigen und flüssigen Bestandteile, die den Wärmeaustauscher 12 verlassen, aufgetrennt werden. An den die Flüssigkeiten aufnehmenden Bereich des Abscheidebehälters 13 schließt sich eine Destillationskolonne 14 an. Dort werden das Acrolein als gasförmiger Bestandteil über Kopf und die Hochsieder, die auch meist Glycerin enthalten, im Sumpf abgetrennt. Die Hochsieder und das Glycerin wird aus dem Sumpf der Destillationskolonne ausgeschleust und das Glycerin wieder dem Dehydratisierungsreaktor 2 zugeführt und die als "HS" gekennzeichneten Hochsieder einer weiteren Verwendung zugeführt. Das gasförmig über Kopf die Destillationskolonne 14 verlassene Acrolein wird mit für die Gasphasenoxidation notwendigen Mengen an Luft und Wasser dem Gasphasenoxidationsreaktor 3 zugeführt. Das bei der Gasphasenoxidation entstehende acrylsäurehaltige Gasgemisch wird sodann der Quencheinheit 4 zugeleitet, dort entsprechend aufbereitet, so dass in der Aufarbeitungseinheit 5 Acrylsäure in gewünschter Reinheit erhalten wird. Diese kann, sofern eine Polymerisation erfolgen soll, der Polymerisationseinheit 6 zugeführt werden.

In dem in Fig. 3 dargestellten Ausschnitt B des Oberbereichs 16 des Dehydratisierungsreaktors 2 ist eine kornisch ausgebildete Verjüngung 17 vorgesehen, die mit dem in Richtung des Gasphasenoxidationsreaktors führenden Auslass 18 abschließt. Außerdem ist eine Ausgestaltung der erfindungsgemäßen Vorrichtung für den Betrieb mit Feststoffkatalysator 19 dargestellt. Dieser wird, insbesondere wenn dieser in Form von Pellets vorliegt, durch eine Feststoffkatalysatoraufnahme 20 gehalten.

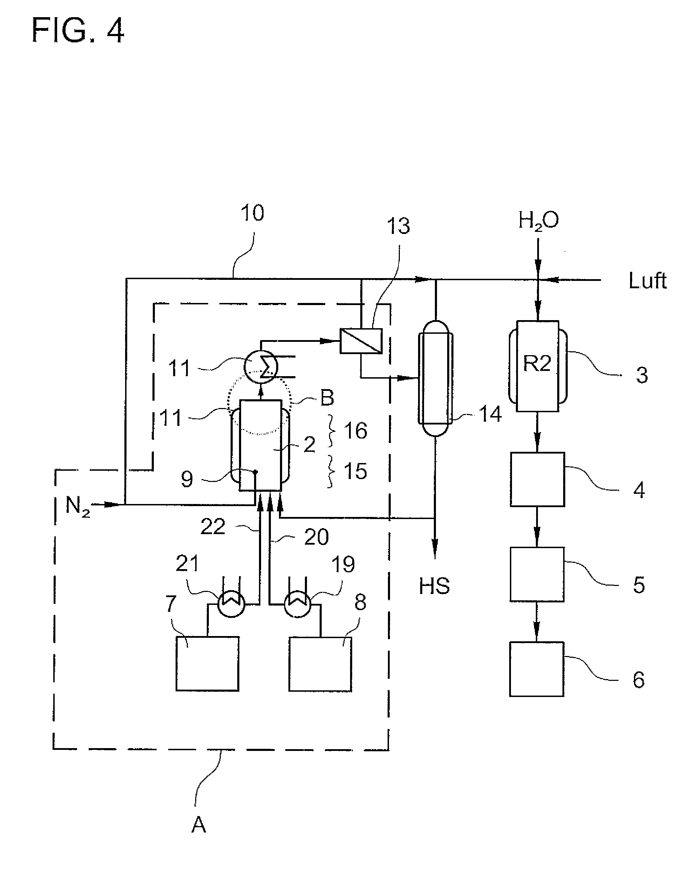

In Fig. 4 wird eine weitere Ausgestaltung des in Fig. 2 dargestellten Dehydratisierungseinheit gefolgt von einer Gasphasenoxidationseinheit mit folgenden Unterschieden gezeigt. Bis auf die nachfolgend dargestellten Unterschiede gelten die Ausführungen zu Fig. 2 auch hier. Im Unterschied zu Fig. 2 wird der Flüssigkatalysator aus dem Flüssigkatalysatorbehälter 8 über einen Katalysatorwärmetauscher 19, der in einer Katalysatorleitung 20 vorgesehen ist und in dem der Katalysator vorgewärmt werden kann, getrennt von dem Edukt in den Dehydratisierungsreaktor 2 eingespeist. Das Edukt wird über einen Eduktwärmetauscher 21 erwärmbar mittels der Eduktleitung 22 ebenfalls separat von dem Katalysator in den Dehydratisierungsreaktor 2 eingespeist. Durch diese Maßnahme kommt es erst in dem Dehydratisierungsreaktor zu einer Vermischung von Edukt und Flüssigkatalysator.

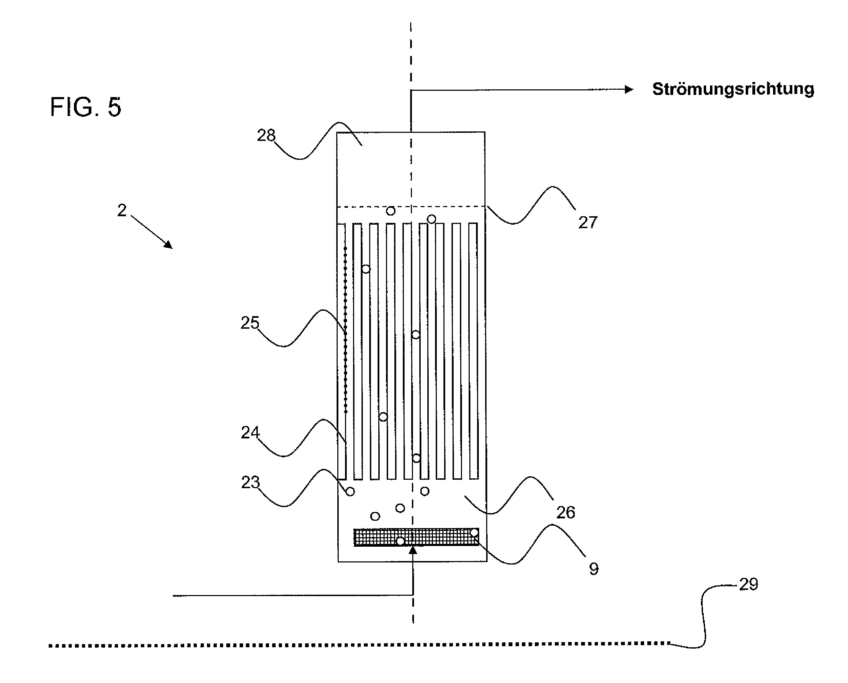

Figur 5 beschreibt den Längsschnitt aus einem Dehydratisierungsreaktor 2, in dem Gasblasen durch einen Gasblasenerzeuger 9 erzeugt werden. Hierbei sollte der Gasblasenerzeuger 9 so ausgestaltet sein, dass eine möglichst gleichmäßige Anströmung des oberhalb des Gasblasenerzeugers 9 vorgesehenen, durchströmbaren Einbau 24 mit den Gasblasen 23 erfolgt. Die durch den Gasblasenerzeuger 9 erzeugten Gasblasen 23 durchwandern den hier als Rohrbündel ausgestalteten durchströmbaren Einbau 24, und können durch Bremsbereiche 25, die vorliegend als aufgeraute Oberflächen der Rohrinnenseiten des Rohrbündelrohrs dargestellt sind, durch Adhäsion verlangsamt werden. Außerdem bestimmt der Querschnitt der Rohre die Durchschnittsgröße der den durchströmbaren Einbau durchwandernden Gasblasen, die den durchströmbaren Einbau 24 nur in der Flüssigphase 26 unterhalb der Phasengrenze 27 vor dem Eintritt in die Gasphase 28 verlassen. Der Dehydratisierungsreaktor ist mit den aufrecht stehenden Rohren des Rohrbündels als durchströmbarer Einbau 24 auf einen Boden 29 angeordnet.

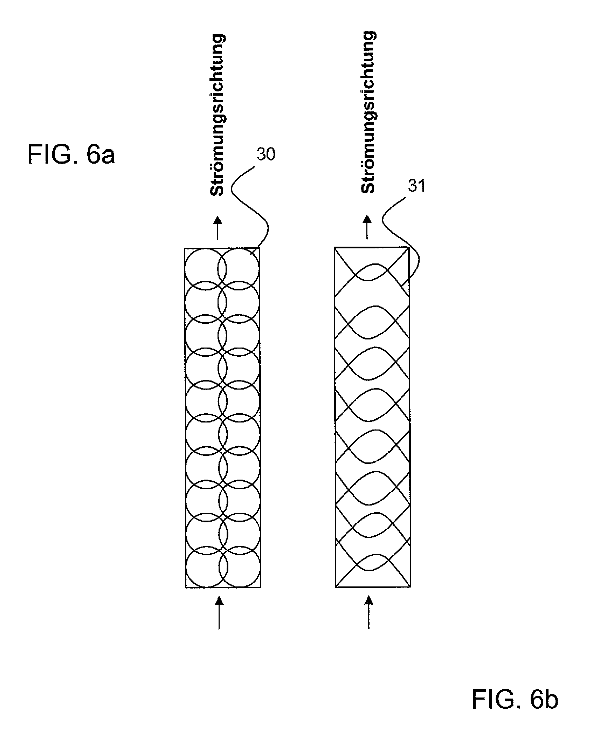

Figuren 6a und 6b zeigen zwei verschiedene Ausgestaltungen von durchströmbaren Einbauten. In Figur 6a sind als durchströmbare Einbauten mit einander verbundene Ringe 30 vorgesehen. In Figur 6b sind miteinander verschlungene Schlaufen 31 als durchströmbare Einbauten gezeigt.

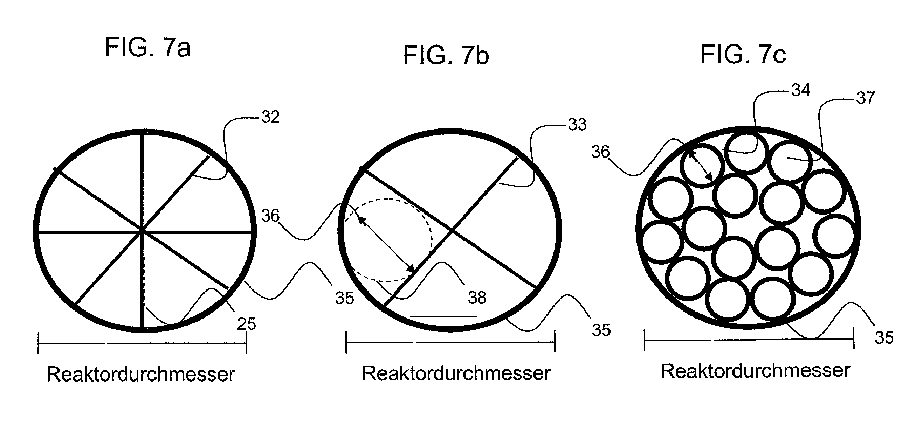

Figuren 7a, b und c zeigen jeweils verschiedene durchströmbare Einbauten in einem Querschnitt durch einen Röhrendehydratisierungsreaktor. In Figur 7a sind sternförmig angeordnete Leitbleche als Sterneinbauten 32 vorgesehen, wobei sich die Leitbleche von der einen zur anderen Seite der Reaktorwand erstrecken. Weiterhin ist ein Bremsbereich 25 beispielhaft als Oberflächenaufrauung auf einem dieser Leitbleche abgebildet. Figur 7b zeigt eine mit Figur 7a vergleichbare Anordnung von Leitblechen, die als Kreuzeinbauten 33 vorgesehen sind. Figur 7c zeigt einen mit in den Figuren 7a und 7b vergleichbaren Aufbau, wobei anstelle der Leitbleche ein Rohrbündeleinbau 34 vorgesehen ist. Die einzelnen Rohre des Rohrbündels zeigen einen Strömungsquerschnitt 36 in einem Schnitt durch den durchströmten Raum 37 eines der Rohre des Rohrbündels. Im Fall der Figur 7b wird beispielhaft die Bestimmung des Strömungsquerschnitts gezeigt. Hierbei wird ein gestrichelt gekennzeichneter Querschnittskreis 38 in die sich aus dem Reaktorquerschnitt ergebenden Fläche der Einbauten gelegt, der an mindestens drei Stellen tangential anliegt. Aus dem zweifachen des Radius dieses Kreises ergibt sich der Strömungsquerschnitt 36 der durch die Einbauten zum Durchlass der Gasblasen gebildet wird.

BEISPIELE

## BEISPIEL 1: FLÜSSIGPHASENDEHYDRATISIERUNG

BEISPIEL 1A:

In einer Vorrichtung gemäß des Ausschnitts A (gestrichelt eingerahmt) der Fig. 2 wurden zur Flüssigphasendehydratisierung eine wässrige (5%ige) Glycerinlösung eingespeist, die zuvor mit Phosphorsäure auf einen pH-Wert von 2,3 gebracht wurde. Die Lösung wurde in dem Reaktor auf 283°C bei 58 bar und einer mittleren Verweilzeit von 9 Minuten erhitzt. Mittels einer Metallfritte wurde Stickstoff in den Reaktor eingesprudelt. Die auf das Reaktorvolumen und Stunde bezogene Stickstoff-Gasbelastung betrug 41 Nml. Der Glycerinumsatz betrug 61 % und die Selektivität zum Acrolein betrug 85,6 %.

BEISPIEL 1B:

In einer Vorrichtung gemäß des Ausschnitts A der Fig. 2 wurden zur Flüssigphasendehydratisierung eine wässrige (5%ige) Glycerinlösung eingespeist, die zuvor mit Phosphorsäure auf einen pH-Wert von 2,3 gebracht wurde. Die Lösung wurde in dem Reaktor auf 285°C bei 61 bar und einer mittleren Verweilzeit von 9 Minuten erhitzt. Mittels einer Metallfritte wurde Stickstoff in den Reaktor eingesprudelt. Die auf das Reaktorvolumen und Stunde bezogene Stickstoff-Gasbelastung betrug 41 N ml. Der Glycerinumsatz betrug 72,1 % und die Selektivität zum Acrolein betrug 74,8 %.

BEISPIEL 1C:

Beispiel 1b wurde zum einen über 310 Stunden ohne Stickstoffspülung und zum anderen über 460 Stunden mit Stickstoffspülung durchgeführt. Bei der Reinigung des Dehydratisierungsreaktors ergaben sich bei der Fahrweise ohne Stickstoff 21 g fester, rußartiger, polymerer Rückstände und bei der Fahrweise mit Stickstoff 8 g dieser Rückstände.

BEISPIEL 1D:

In einer Vorrichtung gemäß des Ausschnitts A (gestrichelt eingerahmt) der Fig. 4 wurden zur Flüssigphasendehydratisierung eine wässrige (5%ige) Glycerinlösung und Phosphorsäure, die jeweils für sich über einen getrennten Wärmetauscher (19, 21), die mit einem Thermoöl mit der Temperatur von 283°C erhitzt wurden, eingespeist, wobei Phosphorsäuremenge so gewählt wurde, dass eine Mischung aus Phosphorsäure und Glycerin einen pH-Wert von 2,3 hat. Die Lösung wurde in dem Reaktor auf 283°C bei 58 bar und einer mittleren Verweilzeit von 9 Minuten erhitzt. Mittels einer Metallfritte wurde Stickstoff in den Reaktor eingesprudelt. Die auf das Reaktorvolumen und Stunde bezogene Stickstoff-Gasbelastung betrug 41 Nml. Der Glycerinumsatz betrug 61 % und die Selektivität zum Acrolein betrug 85 %. Die Wärmetauscher waren nach 400 h voll funktionstüchtig, es lagen keine Verstopfungen vor.

BEISPIEL 1E:

Das Beispiel 1d wurde wiederholt, wobei im Unterschied dazu Phosphorsäure und Glycerinlösung als Gemisch durch einen Wärmetauscher geleitet und dort erhitzt wurden. Der Glycerinumsatz betrug 63,5 % und die Selektivität zum Acrolein betrug 61,3 %.

## BEISPIEL 2: GASPHASENOXIDATION

Im Anschluss an die Acroleinsynthese erfolgte eine Gasphasenoxidation des in den Beispielen 1a bis 1e hergestellten Acroleins in einem handelsüblichen Gasphasenoxidationsreaktor, gefolgt von einer Absorption in Wasser in einer Quencheinheit. Zur Gasphasenoxidation wurde eine dampfförmige, 180 bis 220°C heiße Gasphase mit einer Zusammensetzung von 15 Gew.-% Acrolein, 82 Gew.-% Wasserdampf sowie einem Rest anderer leichter siedender Komponenten analog zu WO 03/051809 A1 zusammen mit 1,5 kg/h vorgeheizter Luft in einen Gasphasenoxidationsrekator eingeleitet, der mit 1,8 l handelsüblichem V-Mo-Multioxidkatalysator befüllt ist.

Die in den Beispielen 1a und 1b erhaltenen Acrylsäurewassergemische wurden in einem auf 0°C temperierten Glasscheidetrichter mit 0,5 Teilen ihres Volumen mit Toluol versetzt. Die Mischung wurde kräftig geschüttelt und über 60 Minuten stehengelassen, um eine Phasentrennung zu ermöglichen. Die beiden so entstandenen Phasen wurden getrennt. Die toluolhaltige Phase wird einer azeotropen Destillation unterzogen und die dabei erhaltene Acrylsäure vor ihrem Einsatz zur Polymerisation nochmals destilliert.

BEISPIEL 3: POLYMERISATION

BEISPIEL 3.0: Herstellung eines nicht oberflächennachvernetzten Polymergebildes - Pulver A

Eine Monomerenlösung bestehend aus 280 g der vorstehend hergestellten und frisch destillierten Acrylsäure, die zu 70 Mol% mit Natronlauge neutralisiert wurde, 466,8 g Wasser, 1,4 g Polyethylenglykol-300-diacrylat und 1,68 g Allyloxypolyethylenglykolacrylsäureester wird durch Spülen mit Stickstoff vom gelösten Sauerstoff befreit und auf die Starttemperatur von 4°C abgekühlt. Nach Erreichen der Starttemperatur wurde die Initiatorlösung (0,1 g 2,2'-Azobis-2-amidinpropandihydrochlorid in 10 g $H_2O$, 0,3 g Natriumperoxydisulfat in 10 g $H_2O$, 0,07 g 30%ge Wasserstoffperoxid-lösung in 1 g $H_2O$ und 0,015 g Ascorbinsäure in 2 g $H_2O$) zugesetzt. Nachdem die Endtemperatur von ca. 100°C erreicht war, wurde das entstandene Gel zerkleinert und bei 150°C 90 Minuten lang getrocknet. Das getrocknete Polymerisat wurde grob zerstoßen, gemahlen und auf ein Pulver mit einer Partikelgröße von 150 bis 850 µm gesiebt. Das Pulver A besitzt eine Retention nach ERT 441.2-02 von 28,8 g/g.

BEISPIEL 3.1: Herstellung eines oberflächennachvernetzten Polymer-gebildes in Gegenwart eines Kieselsäuresols

50 g Pulver A wird mittels eines Krups-Küchenmixers mit einer Lösung aus 0,5 g Ethylencarbonat, 0,42 g Kieselsäuresol (Produkt Levasil® 200 der Bayer AG, Feststoffanteil ca. 30 Gew.-%) und 1,08 g Wasser unter Rühren innig vermischt und anschließend für 30 min. in einem Ofen, der auf 180°C temperiert war, erhitzt. Die Eigenschaften des so erhaltenen Pulvers ergeben sich aus nachfolgender Tabelle.

BEISPIEL 3.2: Herstellung eines oberflächennachvernetzten Polymer-gebildes in Gegenwart eines Kieselsäuresols

50 g Pulver A wird mittels eines Krups-Küchenmixers mit einer Lösung aus 0,5 g Ethylencarbonat, 0,84 g Kieselsäuresol (Produkt Levasil® 200 der Bayer AG, Feststoffanteil ca. 30 Gew.-%) und 0,66 g Wasser unter Rühren innig vermischt und anschließend für 30 min. in einem Ofen, der auf 180°C temperiert war, erhitzt. Die Eigenschaften des so erhaltenen Pulvers ergeben sich aus nachfolgender Tabelle.

Tabelle

| Beispiel | SFC [$10^{-7} \cdot cm^3 \cdot s \cdot g^{-1}$] | AAP (0,7 psi) [g/g] | CRC [g/g] |
|----------|------------------------------|---------------------|-----------|
| 3.1 | 140 | 23,5 | 27 |
| 3.2 | 150 | 23,5 | 27,2 |

BEISPIEL 3.3: Herstellung eines biologisch abbaubaren Polymers

Das im Beispiel 3.1 erhaltene Polymer wurde mit einer wasserlöslichen Weizenstärke (dem Produkt Foralys® 380 der Firma Roquette, Lestrem, Frankreich) im Gewichtsverhältnis Polymer : Stärke von 4 : 1 unter trockenen Bedingungen gemischt und anschließend für 45 Minuten in einem Rollenmixer Typ BTR 10 der Firma Fröbel GmbH, Deutschland, weiter homogenisiert.

BEISPIEL 4: FLÜSSIGPHASENDEHYDRATISIERUNG MIT DURCHSTRÖMBAREN EINBAUTEN

BEISPIEL 4A:

In einem mit einem Strömungsrohr als Dehydratisierungsreaktor ausgelegten Vorrichtung gemäß des Ausschnitts A (gestrichelter Bereich eingerahmt) der Figur 2 wurden eine wässrige (5 %ige) Glycerinlösung und eine wässrige Phosphorsäurelösung (50%), wobei jede Lösung zuvor mittels eines Wärmetauschers auf 250 °C bei 56 bar erhitzt wurde. Die Stoffströme wurden so gewählt, dass in dem Dehydratisierungsreaktor eine Glycerinkonzentration von 7 Gew.-% bezogen auf die gesamte Reaktionsmischung, erreicht wurde. Die Konzentration der Phosphorsäure wurde im Reaktionsgemisch auf einen pH-Wert von 2 eingestellt. Der Reaktor wurde mittels Wärmeträgeröl über eine Außenwandheizung auf eine Reaktorinnentemperatur von 280 °C erhitzt und gehalten. Das Reaktionsgemsich wurde nach Reaktoraustritt auf Raumtemperatur abgekühlt und auf Normaldruck entspannt, so dass Proben mittels Gaschromatographie analysiert werden konnten. Der Reaktor wurde mit einem Reaktionsgemisch von 6 Litern pro Stunde beaufschlagt, das Reaktionsvolumen betrug 2 Liter.

BEISPIEL 4 B:

Der Reaktor aus Beispiel 4a wurde ohne zusätzliche Einbauten betrieben. Bei einem Umsatz von 8 % betrug die Selektivität zum Acrolein 79 %.

BEISPIEL 4C:

In den Reaktor aus Beispiel 4a wurden in das Strömungsrohr Bleche eingebracht, so dass der durch das Strömungsrohr ausgebildete Strömungskanal in 4 Segmente unterteilt wurde (vgl. Fig. 7b). Der Umsatz konnte auf 24 % bei einer Selektivität zum Acrolein von 79 % erhöht werden.

BEISPIEL 4D:

In das Strömungsrohr des Reaktors aus Beispiel 4a wurde ein Rohrbündel mit 16 Röhren vorgesehen. Der Umsatz des so ausgestalteten Reaktors betrug 60 % bei einer Selektivität zum Acrolein von 79 %.

TESTMETHODEN

BESTIMMUNG DES SFC-WERTES

Die Bestimmung der Permeabilität im gequollenen Zustand (*Saline Flow Conductivity* = SFC) erfolgt nach einer in WO-A-95/22356 beschriebenen Methode. In einem Zylinder mit Siebboden werden ca. 0,9 g Superabsorbermaterial (bei Partikeln die gesamte Partikelfraktion) eingewogen und sorgfältig auf der Siebfläche verteilt. Das Superabsorbermaterial lässt man in JAYCO synthetischem Urin 1 Stunde lang gegen einen Druck von 0,7 psi quellen. Nach Erfassung der Quellhöhe des Superabsorbers lässt man bei konstantem hydrostatischem Druck 0,118 M NaCl-Lösung aus einem nivellierten Vorratsgefäß durch die gequollene Gelschicht laufen. Die gequollene Gelschicht ist während der Messung mit einem speziellen Siebzylinder abgedeckt, der eine gleichmäßige Verteilung der 0,118 M NaCl-Lösung oberhalb des Gels und konstante Bedingungen (Messtemperatur 20-25°C) während der Messung bezüglich der Gelbett-Beschaffenheit gewährleistet. Der auf den gequollenen Superabsorber wirkende Druck ist weiterhin 0,7 psi. Mit Hilfe eines Computers und einer Waage wird die Flüssigkeitsmenge, die die Gelschicht als Funktion der Zeit passiert, in Intervallen von 20 Sekunden innerhalb einer Zeitperiode von 10 Minuten erfasst. Die Fliessrate g/s durch die gequollene Gelschicht wird mittels Regressionsanalyse mit Extrapolation der Steigung und Ermittlung des Mittelpunktes auf den Zeitpunkt t=0 der Fließmenge innerhalb der Minuten 2-10 ermittelt. Der SFC-Wert (K) wurde in $cm^3 \cdot s \cdot g^{-1}$ angegeben und wie folgt berechnet:

$$K = \frac{F_s(t=0) \cdot L_0}{r \cdot A \cdot \Delta P_1} = \frac{F_s(t=0) \cdot L_o}{139506}$$

wobei

$F_s(t=0)$      die Fließrate in g/s,
$L_o$      die Dicke der Gelschicht in cm,
$r$      die Dichte derNaCl-Lösung (1,003 $g/cm^3$),
$A$      die Fläche der Oberseite der Gelschicht im Messzylinder (28,27 $cm^2$),
$\Delta P$      der hydrostatische Druck, der auf der Gelschicht lastet (4.920 $dyne/cm^2$), und
$K$      der SFC-Wert ist.

BESTIMMUNG DER RETENTION

Die als CRC bezeichnete Retention wird nach der ERT 441.2-02 bestimmt, wobei "ERT" für "EDANA recommended Test" und "EDANA" für European Disposables and Nonwovens Association" steht.

BESTIMMUNG DER ABSORPTION UNTER DRUCK

Die als AAP bezeichnete Absorption gegen einen Druck von 0,7 psi wird nach der ERT 442.2-02 bestimmt.

BESTIMMUNG DER BIOLOGISCHEN ABBAUBARKEIT

Die Bestimmung der biologischen Abbaubarkeit erfolgt nach dem Sturm-Test gemäß Anhang V zur Richtlinie 67/548/EWG.

Bezugszeichenliste

1 Verseifer
2 Dehydratisierungsreaktor
3 Gasphasenreaktionsreaktor
4 Quencheinheit
5 Aufarbeitungseinheit
6 Polymerisationseinheit
7 Eduktbehälter
8 Flüssigkatalysatorbehälter
9 Gasblasenerzeuger
10 Gasführung
11 Heizvorrichtung
12 Wärmetauscher
13 Abscheidebehälter
14 Destillationskolonne
15 Unterbereich
16 Oberbereich
17 Verjüngung
18 Auslass
19 Katalysatorwärmetauscher
20 Katalysatorleitung
21 Eduktwärmetauscher
22 Eduktleitung
23 Gasblasen
24 durchströmbarer Einbau
25 Bremsbereiche
26 Flüssigphase
27 Phasengrenze

28 Gasphase
29 Boden
30 Ringe
31 Schlaufen
32 Sterneinbauten
33 Kreuzeinbauten
34 Rohrbündeleinbauten
35 Reaktorwand
36 Strömungsquerschnitt
37 Schnitt durch durchströmten Raum
38 Querschnittskreis

**Patentansprüche**

**1.** Verfahren zur Herstellung von Acrylsäure, mindestens die folgenden Schritte aufweisend:

    a. Dehydratisieren von Glycerin zu einem Acrolein aufweisenden Dehydratisierungsprodukt;
    b. Gasphasenoxidation des Dehydratisierungsprodukts unter Erhalt eines Acrylsäure aufweisenden Monomergas;
    c. in Kontakt bringen des Monomergas mit einem Quenchmittel unter Erhalt einer Acrylsäure aufweisenden Quenchphase;

d. Aufarbeiten der Quenchphase unter Erhalt einer Acrylsäure beinhaltenden Monomerphase;

wobei während des Dehydratisierens eine Flüssigphase a1 und eine Gasphase a2 vorliegt, wobei in der Flüssigphase a1 eine Vielzahl von Gasblasen erzeugt werden, wobei das Dehydratisieren mindestens teilweise in der Flüssigphase erfolgt und wobei mindestens ein Teil der Vielzahl von Gasblasen innerhalb der Flüssigphase a1 geleitet oder geteilt wird.

2. Verfahren zur Herstellung eines Polymers durch Polymerisation von Acrylsäure, mindestens die folgenden Schritte aufweisend:

A. Dehydratisieren von Glycerin zu einem Acrolein aufweisenden Dehydratisierungsprodukt;

B. Gasphasenoxidation des Dehydratisierungsprodukts unter Erhalt eines Acrylsäure aufweisenden Monomergas;

C. in Kontakt bringen des Monomergas mit einem Quenchmittel unter Erhalt einer Acrylsäure aufweisenden Quenchphase;

D. Aufarbeiten der Quenchphase unter Erhalt einer Acrylsäure beinhaltenden Monomerphase;

E. Polymerisation der Monomerphase;

wobei während des Dehydratisierens eine Flüssigphase a1 und Gasphase a2 vorliegt, wobei in der Flüssigphase a1 eine Vielzahl von Gasblasen erzeugt werden, wobei das Dehydratisieren mindestens teilweise in der Flüssigphase erfolgt und wobei mindestens ein Teil der Vielzahl von Gasblasen innerhalb der Flüssigphase a1 geleitet oder geteilt wird

3. Verfahren nach einem der vorhergehenden Ansprüche, wobei das Dehydratisieren bei einer Temperatur in einem Bereich von 100 bis 400°C erfolgt.

4. Verfahren nach einem der vorhergehenden Ansprüche, wobei das Dehydratisieren bei einem Druck in einem Bereich von 2 bis 200 bar erfolgt.

5. Verfahren nach einem der vorhergehenden Ansprüche, wobei das Dehydratisieren mit einer mittleren Reaktorverweilzeit in einem Bereich von 1 bis 30 Minuten erfolgt.

6. Verfahren nach einem der vorhergehenden Ansprüche, wobei das durch das Dehydratisieren entstehende Acrolein durch die Vielzahl von Gasblasen aus der Flüssigphase a1 in die Gasphase a2 überführt wird.

7. Verfahren nach einem der vorhergehenden Ansprüche, wobei die Vielzahl von Gasblasen in der Flüssigphase a1 durch Einbringen eines Zusatzgases erzeugt werden.

8. Verfahren nach einem der vorhergehenden Ansprüche, wobei das Glycerin in einer wässrigen Phase eingesetzt wird.

9. Verfahren nach einem der vorhergehenden Ansprüche, wobei das Dehydratisierungsprodukt in einer wässrigen Phase der Gasphasenoxidation zugeführt wird.

10. Verfahren nach einem der vorhergehenden Ansprüche, wobei die Gasphasenoxidation in einem Temperaturbereich von 200 bis 400°C erfolgt.

11. Verfahren nach einem der vorhergehenden Ansprüche, wobei die Menge eines die Vielzahl der Gasblasen erzeugenden Gases variiert wird.

12. Vorrichtung zur Herstellung von Acrylsäure fluidleitend miteinander verbunden beinhaltend

1a. einen Dehydratisierungsreaktor (2),

2a. einen Gasphasenoxidationsreaktor (3),

3a. eine Quencheinheit (4),

4a. eine Aufarbeitungseinheit (5),

wobei der Dehydratisierungsreaktor (2) einen Gasblasenerzeuger (9) beinhaltet und wobei mindestens innerhalb eines Teilbereichs des Dehydratisierungsreaktors (2) mindestens ein durchströmbarer Einbau (24) vorgesehen ist.

13. Vorrichtung zur Herstellung von Polymeren fluidleitend miteinander verbunden beinhaltend

> 1b. einen Dehydratisierungsreaktor (2),
> 2b. einen Gasphasenoxidationsreaktor (3),
> 3b. eine Quencheinheit (4),
> 4b. eine Aufarbeitungseinheit (5),
> 5b. eine Polymerisationseinheit (6),

wobei der Dehydratisierungsreaktor (2) einen Gasblasenerzeuger (9) beinhaltet und wobei mindestens innerhalb eines Teilbereichs des Dehydratisierungsreaktors (2) mindestens ein durchströmbarer Einbau (24) vorgesehen ist.

14. Vorrichtung nach einem der Ansprüche 12 oder 13, wobei der durchstömbare Einbau (24) mindestens in einem Teilbereich stern-, kreuz-, platten-, kugel-, schlaufen-, ring- oder rohrförmig oder in mindestens zwei dieser Formen ausgestaltet ist.

15. Vorrichtung nach Anspruch 12 oder 13, wobei der Dehydratisierungsreaktor (2) in einem Unterbereich (15) den Gasblasenerzeuger aufweist.

**Claims**

1. Process for preparation of acrylic acid, comprising at least the following steps:

> a. dehydrating glycerine to a dehydration product comprising acrolein;
> b. gas phase oxidation of the dehydration product to obtain a monomer gas comprising acrylic acid;
> c. contacting the monomer gas with a quenching agent to obtain a quenched phase comprising acrylic acid;
> d. working up the quenched phase to obtain a monomer phase comprising acrylic acid;

wherein a liquid phase a1 and a gas phase a2 are present during the dehydrating step, wherein a multiplicity of gas bubbles are generated in the liquid phase a1, wherein the dehydrating step is at least partially effected in the liquid phase and wherein at least some of the multiplicity of gas bubbles are conducted or divided within the liquid phase a1.

2. Process for producing a polymer by polymerization of acrylic acid, at least comprising the following steps:

> A. dehydrating glycerine to a dehydration product comprising acrolein;
> B. gas phase oxidation of the dehydration product to obtain a monomer gas comprising acrylic acid;
> C. contacting the monomer gas with a quenching agent to obtain a quenched phase comprising acrylic acid;
> D. working up the quenched phase to obtain a monomer phase comprising acrylic acid;
> E. polymerizing the monomer phase;

wherein a liquid phase a1 and a gas phase a2 are present during the dehydrating step, wherein a multiplicity of gas bubbles are generated in the liquid phase a1, wherein the dehydrating step is at least partially effected in the liquid phase and wherein at least some of the multiplicity of gas bubbles are conducted or divided within the liquid phase a1.

3. Process according to either preceding claim wherein the dehydrating step is effected at a temperature in the range from 100 to 400°C.

4. Process according to any preceding claim wherein the dehydrating step is effected at a pressure in the range from 2 to 200 bar.

5. Process according to any preceding claim wherein the dehydrating step is effected with an average reactor residence time in the range from 1 to 30 minutes.

6. Process according to any preceding claim wherein the acrolein formed by the dehydrating step is transferred from the liquid phase a1 into the gas phase a2 by the multiplicity of gas bubbles.

7. Process according to any preceding claim wherein the multiplicity of gas bubbles is generated in the liquid phase a1 by introduction of an added gas.

8. Process according to any preceding claim wherein the glycerine is used in an aqueous phase.

9. Process according to any preceding claim wherein the dehydration product is fed in an aqueous phase to the gas phase oxidation.

10. Process according to any preceding claim wherein the gas phase oxidation is effected in the temperature range from 200 to 400°C.

11. Process according to any preceding claim wherein the amount of a gas generating the multiplicity of gas bubbles is varied.

12. Apparatus for production of acrylic acid comprising in mutual fluid-conducting communication

    1a. a dehydration reactor (2),
    2a. a gas phase oxidation reactor (3),
    3a. a quenching unit (4),
    4a. a working-up unit (5),

wherein the dehydration reactor (2) comprises a gas bubble generator (9) and wherein at least one internal (24) allowing flow therethrough is provided within at least a sub-region of the dehydration reactor (2).

13. Apparatus for production of polymers comprising in mutual fluid-conducting communication

    1b. a dehydration reactor (2),
    2b. a gas phase oxidation reactor (3),
    3b. a quenching unit (4),
    4b. a working-up unit (5),
    5b. a polymerization unit (6),

wherein the dehydration reactor (2) comprises a gas bubble generator (9) and wherein at least one internal (24) allowing flow therethrough is provided within at least a sub-region of the dehydration reactor (2).

14. Apparatus according to either of Claims 12 and 13 wherein the internal (24) allowing flow therethrough is at least in a sub-region star, cross, plate, sphere, loop, ring or tube shaped or configured in at least two of these shapes.

15. Apparatus according to Claim 12 or 13 wherein the dehydration reactor (2) accommodates the gas bubble generator in a bottom region (15).

**Revendications**

1. Procédé pour la préparation d'acide acrylique, présentant au moins les étapes suivantes :

    a. déshydratation de glycérol en un produit de déshydratation présentant de l'acroléine ;
    b. oxydation en phase gazeuse du produit de déshydratation avec obtention d'un gaz monomère présentant de l'acide acrylique ;
    c. mise en contact du gaz monomère avec un agent de refroidissement brusque avec obtention d'une phase de refroidissement brusque présentant de l'acide acrylique ;
    d. traitement de la phase de refroidissement brusque avec obtention d'une phase monomère contenant de l'acide acrylique ;

où il existe, pendant la déshydratation, une phase liquide a1 et une phase gazeuse a2, une multitude de bulles gazeuses étant générées dans la phase liquide a1, la déshydratation ayant lieu au moins partiellement dans la phase liquide et au moins une partie de la multitude de bulles gazeuses étant guidée ou divisée dans la phase liquide a1.

**2.** Procédé pour la préparation d'un polymère par polymérisation d'acide acrylique, présentant au moins les étapes suivantes :

A. déshydratation de glycérol en un produit de déshydratation présentant de l'acroléine ;

B. oxydation en phase gazeuse du produit de déshydratation avec obtention d'un gaz monomère présentant de l'acide acrylique ;

C. mise en contact du gaz monomère avec un agent de refroidissement brusque avec obtention d'une phase de refroidissement brusque présentant de l'acide acrylique ;

D. traitement de la phase de refroidissement brusque avec obtention d'une phase monomère contenant de l'acide acrylique ;

E. polymérisation de la phase monomère ;

où il existe, pendant la déshydratation, une phase liquide a1 et une phase gazeuse a2, une multitude de bulles gazeuses étant générées dans la phase liquide a1, la déshydratation ayant lieu au moins partiellement dans la phase liquide et au moins une partie de la multitude de bulles gazeuses étant guidée ou divisée dans la phase liquide a1.

**3.** Procédé selon l'une quelconque des revendications précédentes, la déshydratation étant réalisée à une température dans une plage de 100 à 400°C.

**4.** Procédé selon l'une quelconque des revendications précédentes, la déshydratation étant réalisée à une pression dans une plage de 2 à 200 bars.

**5.** Procédé selon l'une quelconque des revendications précédentes, la déshydratation étant réalisée à un temps de séjour moyen dans le réacteur dans une plage de 1 à 30 minutes.

**6.** Procédé selon l'une quelconque des revendications précédentes, l'acroléine formée par la déshydratation étant transférée par la multitude de bulles gazeuses de la phase liquide a1 dans la phase gazeuse a2.

**7.** Procédé selon l'une quelconque des revendications précédentes, la multitude de bulles gazeuses dans la phase liquide a1 étant générée par l'introduction d'un gaz additif.

**8.** Procédé selon l'une quelconque des revendications précédentes, le glycérol étant utilisé dans une phase aqueuse.

**9.** Procédé selon l'une quelconque des revendications précédentes, le produit de déshydratation étant introduit dans une phase aqueuse dans l'oxydation en phase gazeuse.

**10.** Procédé selon l'une quelconque des revendications précédentes, l'oxydation en phase gazeuse étant réalisée dans une plage de température de 200 à 400°C.

**11.** Procédé selon l'une quelconque des revendications précédentes, la quantité d'un gaz générant la multitude de bulles gazeuses étant variée.

**12.** Dispositif pour la préparation d'acide acrylique contenant, en communication fluidique les uns avec les autres,

1a un réacteur de déshydratation (2),

2a. un réacteur d'oxydation en phase gazeuse (3),

3a. une unité de refroidissement brusque (4),

4a. une unité de traitement (5),

le réacteur de déshydratation (2) contenant un générateur de bulles de gaz (9) et au moins un insert (24) pouvant être traversé étant prévu au moins à l'intérieur d'une partie partielle du réacteur de déshydratation (2).

**13.** Dispositif pour la préparation de polymères contenant, en communication fluidique les uns avec les autres,

1b. un réacteur de déshydratation (2),

2b. un réacteur d'oxydation en phase gazeuse (3),

3b. une unité de refroidissement brusque (4),

4b. une unité de traitement (5),
5b. une unité de polymérisation (6),

le réacteur de déshydratation (2) contenant un générateur de bulles de gaz (9) et au moins un insert (24) pouvant être traversé étant prévu au moins à l'intérieur d'une partie partielle du réacteur de déshydratation (2).

14. Dispositif selon l'une quelconque des revendications 12 ou 13, l'insert (24) pouvant être traversé étant réalisé, au moins dans une zone partielle, en forme d'étoile, de croix, de plaque, de bille, de flexible, d'anneau ou de tube ou en au moins deux de ces formes.

15. Dispositif selon la revendication 12 ou 13, le réacteur de déshydratation (2) présentant le générateur de bulles de gaz dans une zone inférieure (15).

# FIG. 1

# FIG. 2

# FIG. 3

# FIG. 4

FIG. 5

Strömungsrichtung

EP 2 061 742 B1

FIG. 6a

Strömungsrichtung

30

Strömungsrichtung

31

FIG. 6b

FIG. 7a      FIG. 7b      FIG. 7c

Reaktordurchmesser     Reaktordurchmesser     Reaktordurchmesser

EP 2 061 742 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- JP 2005213225 A
- WO 2004029016 A
- DE 4238493 C1
- DE 2626887 A
- EP 0534294 A
- US 20020198406 A
- EP 105410 A
- WO 02056812 A
- WO 03051809 A1
- WO 9522356 A

**In der Beschreibung aufgeführte Nicht-Patentliteratur**

- Modern Superabsorbent Polymer Technology. Wiley-VCH, 1998
- *Studies in Surface Science and Catalytics,* 1989, vol. 51
- **K. TANNABE.** *New solid Acids and Bases, their catalytic Properties*
- Modern Superabsorbent Polymer Technology. Wiley-VCH, 1998, 69ff